# FASCICULE DE BREVET EUROPEEN

(11) **EP 2 780 457 B1**
(45) Date de publication et mention de la délivrance du brevet: **04.10.2017**
(21) Numéro de dépôt: 12787764.5
(22) Date de dépôt: 15.10.2012
(51) Int. Cl.: C07K 9/00, C07K 7/08, C07K 14/705, A61K 38/10, C07K 14/47, A61K 38/17

(54) **UTILISATION DE LA FIBROMODULINE ET DU LUMICAN POUR AUGMENTER LA MASSE MUSCULAIRE**
VERWENDUNG VON FIBROMODULIN UND LUMICAN ZUR ERHÖHUNG DER MUSKELMASSE
USE OF FIBROMODULIN AND LUMICAN FOR INCREASING MUSCLE MASS

(30) Priorité: 18.11.2011 FR 1160533
(43) Date de publication de la demande: 24.09.2014
(73) Titulaire: Association Francaise Contre les Myopathies, 75013 Paris (FR)
(72) Inventeur: KICHLER, Antoine, F-91540 Mennecy (FR); SCHERMAN, Daniel, F-75012 Paris (FR)
(74) Mandataire: Cabinet Laurent & Charras
(86) Numéro de dépôt international: PCT/FR2012/052349
(87) Numéro de publication internationale: WO 2013/072587

(56) Documents cités:
- WO-A1-2010/106295
- WO-A2-2004/058988
- WO-A2-2008/030706
- WO-A2-2010/138637
- SIMON GUIRAUD ET AL: "Identification of decorin derived peptides with a zinc dependent anti-myostatin activity", NEUROMUSCULAR DISORDERS, vol. 22, no. 12, 1 décembre 2012 (2012-12-01), pages 1057-1068, XP55057166, ISSN: 0960-8966, DOI: 10.1016/j.nmd.2012.07.002
- S CHAKRAVARTI: "Primary Structure of Human Lumican (Keratan Sulfate Proteoglycan) and Localization of the Gene (LUM) to Chromosome 12q21.3-q22", GENOMICS, vol. 27, no. 3, 10 juin 1995 (1995-06-10), pages 481-488, XP055057428, ISSN: 0888-7543, DOI: 10.1006/geno.1995.1080
- DATABASE Geneseq [Online] 8 février 2001 (2001-02-08), "Human cancer associated protein sequence SEQ ID NO:948.", XP002694351, extrait de EBI accession no. GSP:AAB43503 Database accession no. AAB43503
- TAKAYUKI MIURA ET AL: "Interaction between myostatin and extracellular matrix components", ANIMAL SCIENCE JOURNAL, vol. 81, no. 1, 1 février 2010 (2010-02-01), pages 102-107, XP055029509, ISSN: 1344-3941, DOI: 10.1111/j.1740-0929.2009.00700.x
- KISHIOKA Y ET AL: "Decorin enhances the proliferation and differentiation of myogenic cells through suppressing myostatin activity", JOURNAL OF CELLULAR PHYSIOLOGY, WILEY LISS, NEW YORK, NY, US, vol. 215, no. 3, 1 juin 2008 (2008-06-01), pages 856-867, XP002550992, ISSN: 0021-9541, DOI: 10.1002/JCP.21371 [extrait le 2007-12-28]
- MIURA T ET AL: "Decorin binds myostatin and modulates its activity to muscle cells", BIOCHEMICAL AND BIOPHYSICAL RESEARCH COMMUNICATIONS, ACADEMIC PRESS INC. ORLANDO, FL, US, vol. 340, no. 2, 10 février 2006 (2006-02-10), pages 675-680, XP005226737, ISSN: 0006-291X, DOI: 10.1016/J.BBRC.2005.12.060
- WINOKUR S T ET AL: "Facioscapulohumeral muscular dystrophy (FSHD) myoblasts demonstrate increased susceptibility to oxidative stress", NEUROMUSCULAR DISORDERS, PERGAMON PRESS, GB, vol. 13, no. 4, 1 mai 2003 (2003-05-01), pages 322-333, XP005480819, ISSN: 0960-8966, DOI: 10.1016/S0960-8966(02)00284-5

## Description

### DOMAINE TECHNIQUE

La présente invention vise à augmenter la masse musculaire chez l'homme ou l'animal.

Plus précisément, elle préconise l'utilisation d'une composition comprenant un fragment du lumican de moins de 100 résidus apte à lier la myostatine, pour traiter des conditions pathologiques associées à une fonte musculaire, telles que des dystrophies musculaires, ainsi que l'utilisation non thérapeutique de ladite composition pour augmenter la masse musculaire afin par exemple de compenser la fonte musculaire liée au vieillissement.

### ETAT ANTERIEUR DE LA TECHNIQUE

Les maladies neuromusculaires regroupent des pathologies diverses qui sont généralement associées à une perte de force musculaire transitoire ou permanente. Cette perte de force s'accompagne le plus souvent d'une fonte musculaire, également appelée amyotrophie.

Parmi ces maladies musculaires, les myopathies constituent un groupe important correspondant à des atteintes de la fibre musculaire à proprement parler. Parmi elles, les dystrophies musculaires progressives se caractérisent par une diminution de la force musculaire avec généralement une atrophie des muscles, ainsi que par des anomalies à la biopsie musculaire révélant une modification du tissu. Appartiennent notamment à ce groupe la dystrophie musculaire de Duchenne (ou DMD), la dystrophie musculaire de Becker (ou DMB), ainsi que les myopathies des ceintures.

Pour certaines de ces pathologies, les anomalies génétiques associées ont pu être identifiées. Ainsi, les dystrophies musculaires de Duchenne ou de Becker sont liées à des altérations dans le gène codant la dystrophine, la dystrophie des ceintures de type 2A (LGMD 2A ou calpainopathie) à des altérations dans le gène de la calpaïne 3, les sarcoglycanopathies ou myopathies des ceintures de type LGMD 2C, LGMD 2D, LGMD 2E, LGMD 2F à des défauts dans les gènes des γ-, α-, β- et δ-sarcoglycanes, respectivement (McNally EM, Pytel P, Muscle diseases : the muscular dystrophies. Annu Rev Pathol. 2007 ; Vol 2 :87-109).

Dans ces cas particuliers, différentes approches thérapeutiques, y compris des thérapies géniques, sont en cours de développement mais restent délicates à mettre en oeuvre.

Toutefois et plus généralement dans tous les cas de fonte musculaire, il existe un besoin évident de développer des solutions techniques permettant d'augmenter la masse et/ou le volume musculaire.

Ainsi, le document WO 2005/094446 a identifié des anticorps dirigés contre un épitope localisé entre les résidus 40 et 64 de la myostatine humaine mature susceptibles d'augmenter la masse musculaire. Toutefois, cette stratégie basée sur la reconnaissance de la myostatine par un anticorps n'est pas sans poser de problèmes. Il existe donc le besoin de trouver des solutions alternatives.

Par ailleurs, le document WO 2010/106295 rapporte que la décorine, en particulier sa région N-terminale fixant le zinc, est une solution pour l'augmentation de la masse musculaire. La décorine est une protéine de la matrice extracellulaire appartenant à la famille des SLRP (Small Leucine-Rich Proteoglycan) et plus précisément à la classe I des SLRP.

Au niveau structural, les protéines SLRP ont en commun le fait de posséder des séquences riches en leucine (Leucine-Rich Repeat ou LRR), des résidus cystéine conservés en position N-terminale, et la présence d'au moins une chaîne latérale de glycosaminoglycane (GAG). Cinq classes de SLRP ont pu être définies, notamment sur la base du motif contenant les résidus cystéine (McEwan et al., Structural correlations in the family of small leucine-rich repeat protein and proteoglycans. J. of Struct. Biol. 155(2006) 294-305).

Bien que les protéines de la famille SLRP possèdent des caractéristiques communes, notamment structurales, leur implication fonctionnelle reste totalement imprévisible. Ainsi, il a été démontré dans le document Miura et al. (Decorin binds myostatin and modulates its activity to muscle cells. Biochemical and Biophysical Research Communications 340(2006) 675-680) que le biglycan, autre SRLP de classe I comme la décorine et présentant 57% d'identité avec celle-ci, n'était pas capable de se lier à la myostatine.

Miura et al. (Animal Science Journal, 81(1) (2010) 102-107) ont rapporté que la fibromoduline se lie à la myostatine. Winokur et al. (Neuromuscular Disorders, 13(4) (2003) 322-333) ont montré que l'expression du lumican est réduite dans les myoblastes affectés d'une dystrophie musculaire.

### DESCRIPTION DETAILLEE DE L'INVENTION

La présente invention s'inscrit dans la recherche de nouvelles solutions pour augmenter la masse musculaire.

Ainsi et de manière inattendue, le Demandeur a montré que la fibromoduline et le lumican, deux SRLP de classe II, étaient capables de remplir cette fonction. En outre, le Demandeur a identifié des fragments de ces protéines aptes à assurer cette fonction.

Ainsi et selon un premier aspect, la présente invention vise un peptide comprenant un fragment de la fibromoduline ou du lumican de moins de 100 résidus et apte à lier la myostatine.

Dans le cadre de l'invention, on appelle de manière générique, « fibromoduline » la protéine par exemple décrite par Antonsson, P. et al. (Structure and deduced amino acid sequence of the human fibromodulin gene. Biochim. Biophys. Acta 1174 (2), 204-206 (1993)) ou par Saamanen, A.M., et al. (Murine fibromodulin: cDNA and genomic structure, and age-related expression and distribution in the knee joint. Biochem. J. 355 (PT 3), 577-585 (2001)).

Ainsi la protéine murine correspondante présente la séquence SEQ ID NO: 6 suivante, accessible dans GenBank sous le numéro CAA64454.1 (fibromoduline [Mus musculus]) :

La protéine humaine correspondante présente la séquence SEQ ID NO: 7 suivante, accessible dans GenBank sous le numéro CAA51418.1 (fibromoduline [Homo sapiens]) :

De la même manière, on appelle, de manière générique, « lumican » la protéine décrite par exemple par Grover, J. et al. (The human lumican gene. Organization, chromosomal location, and expression in articular cartilage. J. Biol. Chem. 270 (37), 21942-21949 (1995)) ou Ying, S. et al. (Characterization and expression of the mouse lumican gene. J. Biol. Chem. 272 (48), 30306-30313 (1997)).

La protéine murine correspondante présente la séquence SEQ ID NO: 8 suivante, accessible dans GenBank sous le numéro AAB87767.1 (lumican [Mus musculus]) :

La protéine humaine correspondante présente la séquence SEQ ID NO: 9 suivante, accessible dans GenBank sous le numéro AAA85268.1 (lumican [Homo sapiens]) :

Dans le cadre de l'invention, la fibromoduline ou le lumican peut provenir de tout organisme mais des protéines d'origine humaine ou murine sont privilégiées. Plus généralement et de manière avantageuse, la protéine provient du même organisme que celui dans lequel elle va être administrée. Un des avantages des solutions proposées dans le cadre de la présente invention est que ces protéines sont naturellement présentes chez les mammifères, en particulier chez l'homme, et donc *a priori* non susceptibles d'entraîner des effets secondaires ou des réponses immunes.

Même si ces protéines à l'état naturel présentent une chaîne glycosaminoglycane (GAG), une protéine dépourvue de GAG (GAG-) peut également être mise en oeuvre dans le cadre de l'invention. Celle-ci peut par exemple être obtenue par traitement enzymatique.

Dans le cadre de l'invention, on entend par peptide ou polypeptide, une molécule comportant moins de 100 résidus ou acides aminés, avantageusement moins de 50, voire moins de 40 acides aminés, voire moins de 35 ou même moins de 30 acides aminés.

Selon une première définition, le peptide visé par l'invention est caractérisé par sa capacité à lier la myostatine. Cette liaison peut être évaluée par différentes méthodes, directes ou indirectes, connues de l'homme du métier : par résonance des plasmons de surface (Miura *et al.,* 2006) ; à l'aide d'un plasmide codant pour un gène rapporteur tel que la luciférase et contenant des séquences appelées CAGA (plasmide appelé ici p(CACA)12-Luc) (Dennler S, Itoh S, Vivien D, ten Dijke P, Huet S, Gauthier JM. Direct binding of Smad3 and Smad4 to critical TGF beta-inducible elements in the promoteur of human plasminogen activator inhibitor-type 1 gene. Embo J. 1998;17:3091-100 ; Kurisaki K, Kurisaki A, Valcourt U, Terentiev AA, Pardali K, Ten Dijke P, et al. Nuclear factor YY1 inhibits transforming growth factor beta- and bone morphogenetic protein-induced cell differentiation. Mol Cell Biol. 2003;23:4494-510) ; par la mise en oeuvre d'un test ELISA (voir les exemples de réalisation ci-dessous) ; ...

De manière notoire, ces deux protéines SLRP constituent une première sous-famille de la classe II qui présente, dans le domaine N-terminal, une région consensus riche en cystéines, de séquence CX₃CXCX₉C (C représentant un résidu cystéine et X un acide aminé ; en d'autres termes : une cystéine suivi de 3 aa quelconques puis une cystéine séparée par un aa d'une autre cystéine, elle-même séparée de 9 aa de la dernière cystéine).

Avantageusement et en rapport avec la fibromoduline, un peptide visé comprend la séquence CPQECDCPPNFPTAMYC (SEQ ID NO : 1). En d'autres termes, le peptide comprend un fragment de la fibromoduline correspondant aux résidus 76 à 92 de la fibromoduline murine (SEQ ID NO : 6).

Selon un mode de réalisation privilégié, le peptide comprend ou est constitué de la séquence PPPEPRD**CPQECDCPPNFPTAMYC**DNRNLKYLP (SEQ ID NO : 2). En d'autres termes, le peptide comprend ou est constitué d'un fragment de la fibromoduline correspondant aux résidus 69 à 101 de la fibromoduline murine (SEQ ID NO : 6).

Le peptide correspondant aux résidus 69 à 101 de la fibromoduline humaine (SEQ ID NO : 7) présente la séquence SEQ ID NO : 10 suivante : SPPDPRD**CPQECDCPPNFPTAMYC**DNRNLKYLP

Avantageusement et en rapport avec le lumican, un peptide visé comprend la séquence CAPECNCPHSYPTAMYC (SEQ ID NO : 4) correspondant aux résidus 37 à 53 du lumican murin (SEQ ID NO : 8) ou la séquence CAPECNCPESYPSAMYC (SEQ ID NO :11) correspondant aux résidus 37 à 53 du lumican humain (SEQ ID NO : 9).

Selon un mode de réalisation privilégié, le peptide comprend ou est constitué de la séquence YGQISPN**CAPECNCPHSYPTAMYC**DDLKLKSVP (SEQ ID NO : 5) correspondant aux résidus 30 à 62 du lumican murin (SEQ ID NO : 8) ou de la séquence YGQSSPN**CAPECNCPESYPSAMYC** DELKLKSVP (SEQ ID NO : 12) correspondant aux résidus 30 à 62 du lumican humain (SEQ ID NO : 9).

Les peptides décrits ci-dessus sont définis par leur séquence et leur capacité à lier la myostatine. Le reste de la séquence du peptide peut correspondre aux acides aminés juxtaposant le fragment identifié dans le contexte de la protéine native, auquel cas le peptide est constitué d'un fragment de la fibromoduline ou du lumican à proprement parler. Alternativement, il peut s'agir d'une séquence hétérologue, c'est-à-dire qui ne juxtapose pas le fragment dans la protéine native (par exemple ajout d'une ou plusieurs séquences LRR), voire qui n'est pas issue de ces protéines (par exemple séquences TAG), auquel cas le peptide est un variant peptidique ou peptide chimère.

Par ailleurs, un tel peptide peut subir des modifications chimiques, telles que le greffage d'une biotine (biotinylation), ou toute autre modification qui n'altère pas ses propriétés de liaison à la myostatine (couplage de motif polyéthylèneglycol, incorporation d'acides aminés non naturels, phosphorylation, méthylation, etc...).

Ainsi, l'invention concerne :
- une composition comprenant un fragment du lumican de moins de 100 résidus et apte à lier la myostatine ou un fragment de fibromoduline de moins de 100 résidus et apte à lier la myostatine pour lutter contre la fonte musculaire, et donc son utilisation dans le traitement des pathologies associées à une fonte musculaire ;
- l'utilisation non thérapeutique d'une composition comprenant un fragment du lumican de moins de 100 résidus et apte à lier la myostatine ou un fragment de fibromoduline de moins de 100 résidus et apte à lier la myostatine pour augmenter la masse musculaire.

Dans le cadre de l'invention, le terme « masse musculaire » pourrait indifféremment être remplacé par poids ou volume musculaire.

Il existe un certain nombre de conditions dans lesquelles la fonte musculaire se produit.

Il peut tout d'abord s'agir de conditions pathologiques, en particulier dans le cas des maladies neuromusculaires. La fonte musculaire est une conséquence directe des maladies neuromusculaires et il s'agit alors d'une approche compensatrice. Dans ce cadre, la myopathie de Duchenne est une pathologie particulièrement visée mais toutes les formes de maladies neuromusculaires, en particulier les dystrophies musculaires, peuvent être traitées.

En outre, la cachexie ou marasme est également un état pathologique visé par la présente invention. Cet état se caractérise par une maigreur extrême, en particulier au niveau musculaire, provoquée par une longue maladie ou un apport protéinique ou calorique insuffisant.

Cet état pathologique est notamment observé dans les cas de maladies chroniques telles que le cancer ou le SIDA ou chez les personnes souffrant soit d'insuffisance cardiaque - il y a une atrophie des muscles squelettiques chez 68% des patients -, soit d'incontinence urinaire.

Sans être considérées comme pathologiques à proprement parler, certaines situations sont associées à une fonte musculaire : vieillissement, immobilisation prolongée.... Il existe donc là encore un intérêt à augmenter la masse musculaire.

En outre et notamment dans le domaine agro-alimentaire, l'invention offre la possibilité d'augmenter la production de viande animale. L'utilisation de la fibromoduline ou du lumican, ou du moins d'un fragment actif de ces protéines, présente donc un intérêt tout particulier chez l'animal.

La mise en oeuvre d'un peptide, en lieu et place de la protéine correspondante, présente des avantages certains, notamment au niveau de sa production mais également concernant le risque éventuel d'interférences indésirables *in vivo.* Toutefois, pour la première fois et de manière inattendue, il est montré dans le cadre de la présente invention que le lumican pouvait également être mis en oeuvre avec succès dans les applications susmentionnées.

Sont également visés par la présente invention les dérivés actifs (ou équivalents fonctionnels) des fragments de fibromoduline ou de lumican. L'activité visée que doivent posséder ces dérivés concerne la capacité à lier la myostatine et/ou la capacité à augmenter la masse musculaire, lesquelles sont aisément évaluées grâce à la mise en oeuvre des tests décrits dans la présente demande ou par ailleurs décrits dans la littérature.

En pratique, les dérivés présentent avantageusement 50% d'identité avec l'une des séquences SEQ ID NO : 1 à 2, 4 à 5 et 10 à 12, encore plus avantageusement 60, 70%, 80%, 90% voire 95% d'identité, et conservent leur capacité à lier la myostatine.

Les protéines ou peptides décrits dans l'invention peuvent également se présenter sous forme de protéine/peptide fusion ou protéine/peptide chimérique, avec un autre groupement (fragment protéique ou autre) à leur extrémité N ou C-terminale. Ceux-ci peuvent par exemple, mais de façon non limitative, augmenter le temps de résidence de la protéine ou du peptide dans l'organisme. De telles associations peuvent aussi bien être obtenues à partir d'un ADNc recombiné que par liaison chimique des 2 parties.

La présente invention repose donc sur un apport exogène d'un fragment au moins de fibromoduline ou de lumican. De fait, la composition visée par l'invention comprend soit la protéine ou le peptide en tant que tel, soit un système de production de la protéine ou du peptide.

Concernant la protéine, il peut aussi bien s'agir de la protéine native, purifiée à partir d'un organisme produisant naturellement cette protéine, ou bien d'une protéine recombinante produite à partir d'un quelconque des systèmes de synthèse disponibles et connus de l'homme du métier. Un peptide selon l'invention peut être synthétisé chimiquement ou produit à partir d'un système de synthèse cellulaire, acellulaire, ....

Alternativement, une séquence d'acides nucléiques codant pour ladite protéine ou ledit peptide est placée dans un système d'expression, avantageusement sous le contrôle d'un promoteur dans un vecteur. Après introduction dans l'organisme, la protéine ou le peptide est produit *in vivo.* Le transfert des acides nucléiques (ADN ou ARN) peut se faire soit avec des approches virales de transfert de gènes (par exemple virus adéno-associé ou AAV), soit avec des approches non virales (par exemple par simple injection intramusculaire d'un plasmide). Pour la protéine, un ADN génomique peut avoir un intérêt puisque dans certains cas, la présence des introns stabilise l'ARNm préépissé et améliore sa stabilité dans le noyau et son exportation, ce qui conduit à une meilleure expression protéique.

Les protéines et peptides d'intérêt décrits dans l'invention peuvent donc être fournis sous forme d'acides nucléiques, notamment ADN ou ARN, en particulier codant pour les séquences SEQ ID NO : 1, 2, 4, 5, 10, 11 et 12.

Selon un autre mode de réalisation privilégié, la composition visée par l'invention comprend également du zinc, par exemple sous forme de chlorure de zinc, avantageusement à une concentration comprise entre 1 et 50 µM, voire égale à 15 µM.

Une telle composition peut en outre contenir tout composé ou excipient acceptable, notamment pharmaceutique. La voie d'administration peut aussi bien être intramusculaire qu'intraveineuse, voire sous-cutanée, intrapéritonéale ou orale.

Pour favoriser la prise de greffe de cellules précurseurs ou cellules souches, il peut être avantageux d'associer l'administration de la composition selon l'invention à la greffe de cellules (myoblastes, cellules souches, ....). Cette administration peut se faire de manière simultanée ou décalée dans le temps.

Il peut aussi être avantageux de combiner une thérapie génique, destinée au traitement d'une maladie neuromusculaire, avec l'administration de la composition selon l'invention. Ainsi et selon un mode de réalisation privilégié, un gène thérapeutique est associé au traitement par ladite composition. L'administration des 2 traitements peut se faire de manière simultanée ou décalée dans le temps.

Les effets avantageux des fragments de fibromoduline ou de lumican identifiés dans le cadre de la présente invention se traduisent par une augmentation du volume (ou masse ou poids) musculaire. Ces effets positifs peuvent être observés pour les différents muscles squelettiques, aussi bien chez un organisme atteint d'une pathologie affectant sa masse musculaire que chez un individu sain. A priori, aucun effet secondaire, ni aucune réaction immunologique n'est à déplorer.

Par ailleurs, la présente invention met en lumière le fait que d'autres protéines de la matrice extracellulaire de la famille des SLRP, en dehors de la décorine déjà décrite, sont susceptibles de lier la myostatine et d'avoir un impact positif sur la masse musculaire.

Ainsi, ces protéines et les fragments de celles-ci, aptes à lier la myostatine, sont également décrits:

### 1/ classe I des SLRP :

Une protéine et un peptide de classe I, comprenant une séquence CX₃CXCX₆C (C représentant un résidu cystéine et X un acide aminé ; en d'autres termes : une cystéine suivi de 3 aa quelconques puis une cystéine séparée par un aa d'une autre cystéine, elle-même séparée de 6 aa de la dernière cystéine) et apte à fixer la myostatine, sont visés. Avantageusement, il ne s'agit pas de la décorine ni d'un fragment de la décorine.

En particulier, il peut s'agir de l'asporine (séquences murine et humaine SEQ ID NO : 13 et 14, respectivement) ou un peptide comprenant la séquence CPFGCQCYSRVVHC (SEQ ID NO: 15) et plus précisément comprenant ou constitué de la séquence PVNPFFPFDLFPT**CPFGCQCYSRVVHC**SDLGLTSVP (SEQ ID NO : 16) correspondant au fragment 55-90 de l'asporine murine ou de la séquence PRSHFFPFDLFPM**CPFGCQCYSRVVH**CSDLGLTSVP (SEQ ID NO : 17) correspondant au fragment 62-97 de l'asporine humaine.

La protéine murine correspondante présente la séquence SEQ ID NO: 13 suivante, accessible dans GenBank sous le numéro AAI45905.1 (Asporin [Mus musculus]) :

La protéine humaine correspondante présente la séquence SEQ ID NO: 14 suivante, accessible dans GenBank sous le numéro AAK35161.1 (asporin precursor [Homo sapiens]):

### 2/ classe II des SLRP :

Une protéine et un peptide de classe II, comprenant une séquence CX₃CXCX₉C (C représentant un résidu cystéine et X un acide aminé ; en d'autres termes : une cystéine suivi de 3 aa quelconques puis une cystéine séparée par un aa d'une autre cystéine, elle-même séparée de 9 aa de la dernière cystéine) et apte à fixer la myostatine, sont visés.

En dehors de la fibromoduline et du lumican, il peut s'agir de l'osteoadhérine (séquences murine et humaine SEQ ID NO : 18 et 19, respectivement) ou un peptide comprenant la séquence d'origine murine CAKECFCPTNFPTSMYC (SEQ ID NO : 20) ou la séquence d'origine humaine CVSECFCPTNFPSSMYC (SEQ ID NO : 21) et plus précisément comprenant ou constitué de la séquence YGVPFYNNILG**CAKECFCPTNFPTSMYC**DNRKLKTIP (SEQ ID NO: 22) correspondant au fragment 51-87 de l'osteoadhérine murine ou de la séquence YGVPFHQYTLG**CVSECFCPTNFPSSMYC**DNRKLKTIP (SEQ ID NO: 23) correspondant au fragment 51-87 de l'osteoadhérine humaine.

La protéine murine correspondante présente la séquence SEQ ID NO: 18 suivante, accessible dans NCBI sous le numéro NP_036180.1 (osteoadherin (osteomodulin) precursor [Mus musculus]):

La protéine humaine correspondante présente la séquence SEQ ID NO: 19 suivante, accessible dans Swiss-Prot sous le numéro Q99983.1 (human Osteoadherin; Precursor):

Alternativement, il peut s'agir du PRELP (séquences murine et humaine SEQ ID NO : 24 et 25, respectivement) ou un peptide comprenant la séquence CYCPPDFPSALYC (SEQ ID NO : 26) et plus précisément comprenant ou constitué de la séquence PPSVFPDCPRE**CYCPPDFPSALYC**DSRNLRRVP (SEQ ID NO: 27) correspondant au fragment 62-94 du PRELP murin ou de la séquence PPSIFPDCPRE**CYCPPDFPSALYC**DSRNLRKVP (SEQ ID NO : 28) correspondant au fragment 66-98 du PRELP humain.

La protéine murine correspondante présente la séquence SEQ ID NO: 24 suivante, accessible dans GenBank sous le numéro AAF72994.2 (PRELP [Mus musculus]) :

La protéine humaine correspondante présente la séquence SEQ ID NO: 25 suivante, accessible dans GenBank sous le numéro CAG47066.1 (PRELP [Homo sapiens]):

### 3/ classe III des SLRP :

Une protéine et un peptide de classe III, comprenant une séquence CX₂CXCX₆C (C représentant un résidu cystéine et X un acide aminé ; en d'autres termes : une cystéine suivi de 2 aa quelconques puis une cystéine séparée par un aa d'une autre cystéine, elle-même séparée de 6 aa de la dernière cystéine) et apte à fixer la myostatine, sont visés.

Il peut s'agir de l'épyphican (séquences murine et humaine SEQ ID NO : 29 et 30, respectivement) ou un peptide comprenant la séquence CLLCTCISTTVYC (SEQ ID NO: 31) et plus précisément comprenant ou constitué de la séquence TNEDFPT**CLLCTCISTTVYC**DDHELDAIP (SEQ ID NO : 32) correspondant au fragment 111-139 de l'épyphican murin ou humain.

La protéine murine correspondante présente la séquence SEQ ID NO: 29 suivante, accessible dans NCBI sous le numéro NP_031910.1 (epiphycan precursor [Mus musculus]):

La protéine humaine correspondante présente la séquence SEQ ID NO: 30 suivante, accessible dans GenBank sous le numéro AAH30958.1 (Epiphycan [Homo sapiens]):

Alternativement, il peut s'agir de l'osteoglycine (séquences murine et humaine SEQ ID NO: 33 et 34, respectivement) ou un peptide comprenant la séquence CLLCVCLSGSVYC (SEQ ID NO : 35) et plus précisément comprenant ou constitué de la séquence KENDEMPT**CLLCVCLSGSVYC**EEVDIDAVP (SEQ ID NO : 36) correspondant au fragment 86-115 de l'osteoglycine murine ou humaine.

La protéine murine correspondante présente la séquence SEQ ID NO: 33 suivante, accessible dans GenBank sous le numéro AAH21939.1 (Osteoglycin [Mus musculus]) :

La protéine humaine correspondante présente la séquence SEQ ID NO: 34 suivante, accessible dans GenBank sous le numéro AAH37273.1 (Osteoglycin [Homo sapiens]):

Enfin, il peut s'agir de l'opticine (séquences murine et humaine SEQ ID NO : 37 et 38, respectivement) ou un peptide comprenant la séquence CLVCVCLGSSVYC (SEQ ID NO : 39) et plus précisément comprenant ou constitué de la séquence NSQSSHGLPT**CLVCVCLGSSVYC**DDADLENIP (SEQ ID NO : 40) correspondant au fragment 114-145 de l'opticine murine ou de la séquence SSQPNHGLPT**CLVCVCLGSSVYC**DDIDLEDIP (SEQ ID NO : 41) correspondant au fragment 118-149 de l'opticine humaine.

La protéine murine correspondante présente la séquence SEQ ID NO: 37 suivante, accessible dans GenBank sous le numéro AAL78287.1 (opticin [Mus musculus]) :

La protéine humaine correspondante présente la séquence SEQ ID NO: 38 suivante, accessible dans GenBank sous le numéro AAL78286.1 (opticin [Homo sapiens]):

### 4/ classe IV des SLRP :

Une protéine et un peptide de classe IV, comprenant une séquence Cx₃CXCX₆₋₁₇C (C représentant un résidu cystéine et X un acide aminé ; en d'autres termes : une cystéine suivi de 3 aa quelconques puis une cystéine séparée par un aa d'une autre cystéine, elle-même séparée par 6 à 17 aa de la dernière cystéine) et apte à fixer la myostatine, sont visés.

Il peut s'agir de la chondroadhérine (séquences murine et humaine SEQ ID NO : 42 et 43, respectivement) ou un peptide comprenant la séquence d'origine murine CPQNCHCHGDLQHVIC (SEQ ID NO : 44) ou la séquence d'origine humaine CPQNCHCHSDLQHVIC (SEQ ID NO : 45) et plus précisément comprenant ou constitué de la séquence LAILLPALAA**CPQNCHCHGDLQHVIC**DKVGLQKIP (SEQ ID NO : 46) correspondant au fragment 12-46 de la chondroadhérine murine ou de la séquence LAGLLPALAA**CPQNCHCHSDLQHVIC**DKVGLQKIP (SEQ ID NO : 47) correspondant au fragment 13-47 de la chondroadhérine humaine.

La protéine murine correspondante présente la séquence SEQ ID NO: 42 suivante, accessible dans GenBank sous le numéro AAC39963.1 (chondroadherin [Mus musculus]) :

La protéine humaine correspondante présente la séquence SEQ ID NO: 43 suivante, accessible dans GenBank sous le numéro AAC13410.1 (chondroadherin [Homo sapiens]) :

Alternativement, il peut s'agir de la nyctalopine (séquences murine et humaine SEQ ID NO : 48 et 49, respectivement) ou un peptide comprenant la séquence d'origine murine CLRACPAACTCSHVERGCSVRC (SEQ ID NO: 50) ou la séquence d'origine humaine CARACPAACACSTVERGCSVRC (SEQ ID NO : 51) et plus précisément comprenant ou constitué de la séquence YTRATEA**CLRACPAACTCSHVERGCSVRC**DRAGLQRVP (SEQ ID NO : 52) correspondant au fragment 15-52 de la nyctalopine murine ou de la séquence SAWAVGA**CARACPAACACSTVERGCSVRC**DRAGLLRVP (SEQ ID NO : 53) correspondant au fragment 20-57 de la nyctalopine humaine.

La protéine murine correspondante présente la séquence SEQ ID NO: 48 suivante, accessible dans GenBank sous le numéro AAM47034.1 (nyctalopin [Mus musculus]) :

La protéine humaine correspondante présente la séquence SEQ ID NO: 49 suivante, accessible dans GenBank sous le numéro AAG42685.1 (nyctalopin [Homo sapiens]) :

Alternativement, il peut s'agir du tsukushi (séquences murine et humaine SEQ ID NO : 54 et 55, respectivement) ou un peptide comprenant la séquence d'origine murine CFPGCQCEEETFGLFDSFSLIRVDC (SEQ ID NO : 56) ou la séquence d'origine humaine CFPGCQCEVETFGLFDSFSLTRVD (SEQ ID NO : 57) et plus précisément comprenant ou constitué de la séquence RVQTTRP**CFPGCQCEEETFGLFDSFSLIRVDC**SSLGPHIVP (SEQ ID NO : 58) correspondant au fragment 14-54 du tsukushi murin ou de la séquence GAQTTRP**CFPGCQCEVETFGLFDSFSLTRVDC**SGLGPHIMP (SEQ ID NO: 59) correspondant au fragment 13-53 du tsukushi humain.

La protéine murine correspondante présente la séquence SEQ ID NO: 54 suivante, accessible dans GenBank sous le numéro BAD98727.1 (Tsukushi [Mus musculus]) :

La protéine humaine correspondante présente la séquence SEQ ID NO: 55 suivante, accessible dans Swiss-Prot sous le numéro Q8WUA8.3 (Tsukushi [homo sapiens]) :

### 5/ classe V des SLRP :

Une protéine et un peptide de classe V, comprenant une séquence CX₃₋₄CXCX₇C (C représentant un résidu cystéine et X un acide aminé ; en d'autres termes : une cystéine suivi de 3 ou 4 aa quelconques puis une cystéine séparée par un aa d'une autre cystéine, elle-même séparée de 7 aa de la dernière cystéine) et apte à fixer la myostatine, sont visés.

Il peut s'agir du podocan (séquences murine et humaine SEQ ID NO : 60 et 61, respectivement) ou un peptide comprenant la séquence CPRDCACSQEGVVDC (SEQ ID NO : 62) et plus précisément comprenant ou constitué de la séquence PGPATVD**CPRDCACSQEGVVDC**GGIDLREFP (SEQ ID NO : 63) correspondant au fragment 62-92 du podocan murin ou de la séquence PGPAAVS**CPRDCACSQEGVVDC**GGIDLREFP (SEQ ID NO : 64) correspondant au fragment 61-91 du podocan humain.

La protéine murine correspondante présente la séquence SEQ ID NO: 60 suivante, accessible dans GenBank sous le numéro CAM23596.1 (Podocan [Mus musculus]):

La protéine humaine correspondante présente la séquence SEQ ID NO: 61 suivante, accessible dans GenBank sous le numéro AAP79898.1 (Podocan [Homo sapiens]):

Il peut également s'agir du podocan-like protein 1 (séquences murine et humaine SEQ ID NO : 65 et 66, respectivement) ou un peptide comprenant la séquence d'origine murine CPWRCSCPRDDTVDC (SEQ ID NO : 67) ou la séquence d'origine humaine CPLRCSCPRVDTVDC (SEQ ID NO : 68) et plus précisément comprenant ou constitué de la séquence GDSSQPLPRP**CPWRCSCPRDDTVDC**AGLDLRIFP (SEQ ID NO : 69) correspondant au fragment 29-62 du podocan-like protein 1 murin ou de la séquence GESLQPLLRA**CPLRCSCPRVDTVDC**DGLDLRVFP (SEQ ID NO : 70) correspondant au fragment 36-69 du podocan-like protein 1 humain.

La protéine murine correspondante présente la séquence SEQ ID NO: 65 suivante, accessible dans NCBI sous le numéro NP_001013402.2 (podocan-like protein 1 precursor [Mus musculus]) :

La protéine humaine correspondante présente la séquence SEQ ID NO: 66 suivante, accessible dans GenBank sous le numéro AAH57786.1 (Podocan-like 1 [Homo sapiens]) :

Enfin l'invention peut concerner l'ECM2 (séquences murine et humaine SEQ ID NO : 71 et 72, respectivement) ou un peptide comprenant ou constitué de la séquence AVWSPEP**CTTCLCSNGRVLC**DETECHPKACP (SEQ ID NO : 73) correspondant au fragment 109-139 de l'ECM2 murine ou de la séquence AVWSPEP**CTTCLCSDGRVLC**DETMCHPQRCP (SEQ ID NO : 74) correspondant au fragment 114-144 de l'ECM2 humaine.

La protéine murine correspondante présente la séquence SEQ ID NO: 71 suivante, accessible dans NCBI sous le numéro NP_001012324.1 (extracellular matrix protein 2 precursor [Mus musculus]) :

La protéine humaine correspondante présente la séquence SEQ ID NO: 72 suivante, accessible dans GenBank sous le numéro AAI05959.1 (ECM2 protein [Homo sapiens]) :

La présente demande décrit donc :
- les protéines et peptides tels que définis, ainsi que leurs variants fonctionnels ou actifs ;
- l'utilisation de ces protéines ou peptides pour la détection, la purification et/ou la titration de la myostatine.
- des compositions comprenant ces protéines ou peptides pour leur utilisation dans le traitement des pathologies associées à une fonte musculaire, notamment maladies neuromusculaires, avantageusement les dystrophies musculaires telles que la myopathie de Duchenne, et les cachexies ;
- des compositions comprenant ces protéines ou peptides pour augmenter la masse musculaire, notamment pour compenser la fonte résultant d'une immobilisation ou de la vieillesse, ou chez l'animal ;
- La présence de zinc dans ces compositions ;
- l'administration de ces compositions par voie intramusculaire, intrapéritonéale, sous-cutanée, orale ou intraveineuse ;
- l'association de ces compositions à d'autres traitements, tels que des thérapies géniques et la greffe de cellules.

### EXEMPLES DE REALISATION

L'invention et les avantages qui en découlent ressortiront mieux des exemples de réalisation suivants, à l'appui des figures annexées. Ceux-ci n'ont toutefois aucune portée limitative.

L'invention est illustrée plus avant à l'aide d'un fragment de fibromoduline ou de lumican recombinant de souris, testé *in vitro* pour sa liaison à la myostatine ou injecté par voie intramusculaire dans des souris *mdx* possédant un gène codant une dystrophine altérée et servant de modèle d'étude pour la myopathie de Duchenne.

### LEGENDES DES FIGURES

La figure 1 représente l'inhibition de l'activité de la myostatine par le peptide Fmod murin 69-101 (SEQ ID NO :2) dans les celllules HEK293T *in vitro*, en présence ou en absence de zinc.
La figure 2 représente l'inhibition de l'activité de la myostatine par le peptide Lumican murin 30-62 (SEQ ID NO :5) dans les cellules HEK293T *in vitro*, en présence ou en absence de zinc.
La figure 3 représente l'interaction *in vitro* entre les peptides Fmod 69-101 (SEQ ID NO :2) et Fmod 69-101 muté (SEQ ID NO :3) et la myostatine, en présence ou en absence de zinc.

### I) MATERIELS ET METHODES

### 1. Cassette d'expression pGL3-(CAGA)₁₂-Luc :

La construction plasmidique pGL3-(CAGA)₁₂-firefly luciférase (notée pGL3-(CAGA)₁₂-Luc) obtenue par MTA du Pr. Carl-Henrik Heldin (Ludwig Institute for Cancer Research, Suède) contient 12 copies CAGA de la séquence consensus SBE (Smad Binding Element) et permet indirectement de mesurer la liaison de la myostatine à son récepteur. En effet, lorsque la myostatine se fixe au récepteur Activine IIb, les protéines Smad 3 et 4 sont activées et vont se lier aux 12 copies CAGA. Le promoteur de la cassette pGL3-(CAGA)₁₂-Luc est ainsi activé, ce qui se traduit par une production de luciférase. La liaison myostatine/Activine IIb peut donc être suivie en dosant la luciférase. En conséquence, cet essai peut aussi être utilisé pour mettre en évidence la capacité d'un composé (protéine ou peptide) à empêcher la myostatine à se lier à son récepteur.

### 2. Etude in vitro de l'inhibition de la Myostatine :

La capacité des peptides à inhiber l'activité *in vitro* de la myostatine a été évaluée sur des cellules humaines embryonnaires rénales 293T au moyen de la construction pGL3-(CAGA)₁₂-Luc. Pour cela, 350 000 cellules 293T ont été déposées par puits dans une plaque 24 puits. Après 24 h de culture, les cellules ont été transfectées avec un mélange de 8 µl de PEI (PEI 25 kDa; Sigma) et de 2 µg de plasmide pGL3-(CAGA)₁₂-Luc/puits. A noter que toutes les expériences de transfection ont été réalisées en duplicat. Vingt-quatre heures après transfection, 2,5 nM de myostatine murine recombinante mélangée ou non à différentes quantités de peptides fibromoduline ou lumican ont été ajoutés dans chaque puits. Une incubation préalable de 30 minutes à 37°C a été réalisée pour les conditions regroupant myostatine et l'inhibiteur potentiel. Cette pré-incubation a - sauf indications contraires - toujours été réalisée en présence de 15 µM de ZnCl₂. Vingt-quatre heures après incubation, les cellules ont été lysées, puis récoltées afin de doser l'activité de la luciférase et la quantité de protéine.

Les peptides fibromoduline ou lumican suivants ont été utilisés :
- pour la fibromoduline : Fmod : PPPEPRDCPQECDCPPNFPTAMYCDNRNLKYLP (SEQ ID NO : 2).
- pour le lumican : mLumican : YGQISPNCAPECNCPHSYPTAMYCDDLKLKSVP (SEQ ID NO : 5).

### 3. Mise en évidence de la liaison MSTN/peptides fibromoduline (Fmod)

Le test ELISA (Enzyme Linked ImmunoSorbent Assay) est un test destiné à détecter et/ou doser une protéine présente dans un liquide biologique. Dans la technique de dosage dite "en sandwich", les puits d'une microplaque sont tapissés avec un anticorps de capture capable de lier spécifiquement l'antigène recherché. Lors de cette opération appelée « coating », l'anticorps de capture se fixe au plastique des puits par interaction électrostatique. L'anticorps de capture assure la spécificité du test. La solution à tester est ensuite déposée dans les puits de la microplaque et, si l'antigène recherché est présent, il va se lier spécifiquement à l'anticorps de capture. Un deuxième anticorps, l'anticorps traceur, capable de se lier à l'antigène capturé, est alors ajouté dans le puits et les anticorps traceurs non fixés sont éliminés par rinçage. L'anticorps traceur est couplé à une enzyme catalysant la formation d'un produit coloré.

Le principe de cette technique a été utilisé pour étudier l'interaction directe de la myostatine mature avec les peptides 69-101 Fmod Biotine et 69-101 Fmod Biotine mutée (voir séquences ci-dessous). Pour cela, 3 µl de myostatine à la concentration de 100 ng/µl sont repris dans 50 µl de tampon carbonate et déposés dans chaque puits de la plaque de dosage (R&D Systems, DY990). Après une incubation d'une nuit à 4°C, la plaque est lavée avec 100 µl de PBS-tween (Tween 20 à 0,05 %) puis l'étape de blocage permettant de saturer les sites non spécifiques est réalisée pendant 2 heures à température ambiante avec 100 µl de PBS et 6 % de lait par puits. Cinq lavages successifs avec 200 µl de PBS-tween (Tween 20 à 0,05 %) sont effectués et des quantités croissantes de peptides fibromoduline biotinylés sont ensuite déposées (100 µl final dans du PBS) et la plaque est incubée durant 1 heure à 37°C. Pour le contrôle positif, un anticorps de chèvre anti-myostatine (≠AF788, R&D Systems), qui a été déposé dans les puits contrôles et incubé 1 heure au 1/100^{ième} (concentration de 0,1 mg/ml), a été utilisé. Les puits ont ensuite été lavés 5 fois avec du PBS-tween (Tween 20 à 0,05 %). Pour détecter la liaison des peptides biotinylés à la MSTN, de la streptavidine-HRP (≠N100, Pierce) qui a été utilisée au 1/20 000^{ième}, a été ajouté dans les puits. Pour les points contrôles avec l'anticorps de chèvre anti-myostatine, un anticorps secondaire polyclonal d'âne anti-chèvre marqué à la HRP (≠ab6885, Abcam) a été ajouté (au 1/4000^{ième}). Après 5 lavages, l'ajout du substrat de la peroxydase, le TMB Substrate Reagent Set (Tétraméhylbenzidine, OptEIA, ≠555214), permet l'obtention d'une réaction colorée proportionnelle à la quantité de HRP présente dans le puits. La réaction est stoppée au bout de 20 minutes par ajout d'acide sulfurique 2N puis la plaque est lue à 450 nm sur un spectrophotomètre Discovery HT-R (Bio-TEK).

Composition du tampon : tampon carbonate 0,1 M : 4,2 g NaHCO₃, 1,78 g de Na₂CO dans 500 ml d'H₂0 MilliQ, pH 9,5. Conservation à 4°C.

### Séquences des peptides biotinylés :

- Peptide Biotine- 69-101 mFibromoduline (Fmod)
   ∘ Biotine-PPPEPRDCPQECDCPPNFPTAMYCDNRNLKYLP- *CONH₂* (SEQ ID NO : 2)
- Peptide Biotine- 69-101 mFibromoduline mutée (Fmod mutée)
   ∘ Biotine-PPPEPRDCPQE**A**D**A**PPNFPTAMY**A**DNRNLKYLP- *CONH₂* (SEQ ID NO : 3)

### 4. Expérimentation animale:

### a. Souris

Les souris *mdx* (C57BL/10ScSn-*DmD*^{mdx}/J) proviennent des colonies entretenues à Généthon.

Des souris âgées d'au moins 6 semaines, ont été utilisées. Toutes les expériences ont été réalisées en conformité avec les règles européennes d'éthique concernant l'utilisation des animaux pour la recherche expérimentale.

### b. Anesthésie

Les souris ont été anesthésiées avant chaque injection intramusculaire et avant chaque prélèvement de sang par une injection intrapéritonéale d'un mélange de Kétamine à 100 mg/kg et de Xylazine à 1 mg/kg (0,1 ml/10 g).

### c. Injection intramusculaire

L'injection intramusculaire est réalisée sur animal anesthésié au niveau du muscle tibialis antérieur (TA). Le volume injecté a été de l'ordre de 20 à 35 µl. L'injection est réalisée au milieu du TA Gauche (TAG). Un volume identique de NaCl 150 mM/15 µM ZnCl₂ a été systématiquement injecté dans le tibial antérieur droit (TAD) afin de servir de contrôle.

Après un nombre déterminé de jours après l'injection, les TAD et TAG sont prélevés après sacrifice des souris, pesés puis congelés en vue d'études histologiques.

### d. Préparation de la solution de peptide

Le peptide fibromoduline (Fmod, SEQ ID NO : 2) et lumican (mLumican, SEQ ID NO : 5) ont été dissouts dans de l'eau puis stockés à -80°C. Pour les injections, le protocole de préparation est le suivant : 24-40 heures avant l'injection, la quantité désirée de peptide est prélevée de la solution mère et mélangée avec une solution de chlorure de zinc (ZnCl₂) et de NaCl 150 mM, de manière à avoir une concentration finale de 15 µM en zinc. Le peptide est conservé à +4°C jusqu'au moment de l'injection.

### e. Analyses histologiques

### - Marquage laminine :

Des sections cryostat (8 µm) des muscles traités et témoins sont réalisés suivant les techniques standards. Les lames sont fixées avec du Dakopen (DAKO^{®}, Réf. : S 2002) pendant 10 minutes à l'air libre et ensuite bloquées avec une solution de PBS / sérum de chèvre 10%, pendant 30 min à température ambiante et en chambre humide. L'anticorps lapin anti laminine (DAKO^{®}, Réf. : Z0097) est appliqué sur les lames à une dilution de 1 : 1000 pendant 12h en chambre humide. Les lames sont alors rincées dans du PSB (5 minutes) sous agitation et l'anticorps secondaire (*Kit Envision HRP Rabbit)* est appliqué sur les lames en chambre humide pendant 30 min à température ambiante. Après avoir rincé les lames dans du PB S (5 minutes) sous agitation, la DAB (DAKO^{®}, Réf. : K 3466) est appliquée sur les coupes pendant 2 à 5 minutes à température ambiante et en chambre humide. Les lames sont rincées en renouvellement constant et sont montées sous la hotte chimique. L'analyse des résultats est réalisée à l'aide du logiciel ELLIX.

### - Marquage HPS :

Des sections cryostat (8 µm) des muscles traités et témoins sont réalisés suivant les techniques standards. Les lames sont plongées dans l'hématoxyline de Harris pendant 3 minutes pour être ensuite rincées à l'eau courante. Elles sont ensuite trempées dans l'alcool chlorhydrique, rincées et plongées dans l'eau de Scott pendant une minute. Après rinçage, les lames sont plongées dans la Phloxine pendant 30 secondes, rincées à l'eau courante et trempées dans l'éthanol absolu pendant une minute. Après une exposition au Safran pendant 3 minutes, elles sont rincées à l'éthanol absolu et monté avec la résine Eukitt dont le solvant est le Xylène. L'analyse des résultats est réalisée à l'aide du logiciel CARTHOGRAPH.

### II) RESULTATS

### 1/ Inhibition de l'activité de la myostatine par les peptides Fmod 69-101 (SEQ ID NO : 2) et mLum 30-62 (SEQ ID NO: 5) dans les cellules HEK293T in vitro :

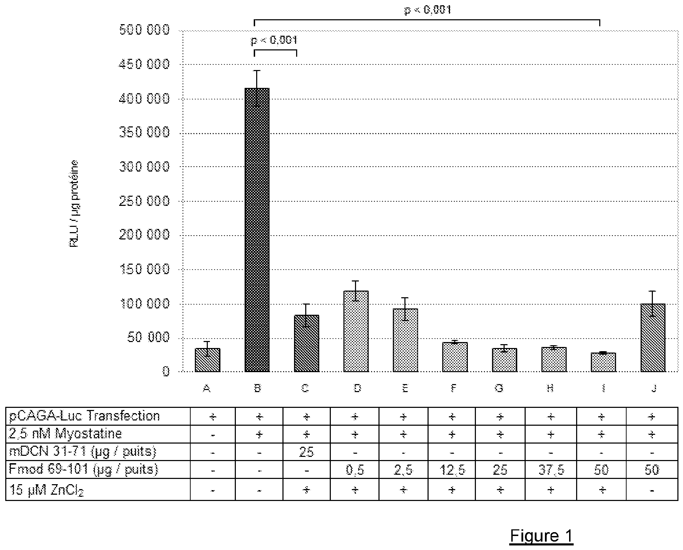

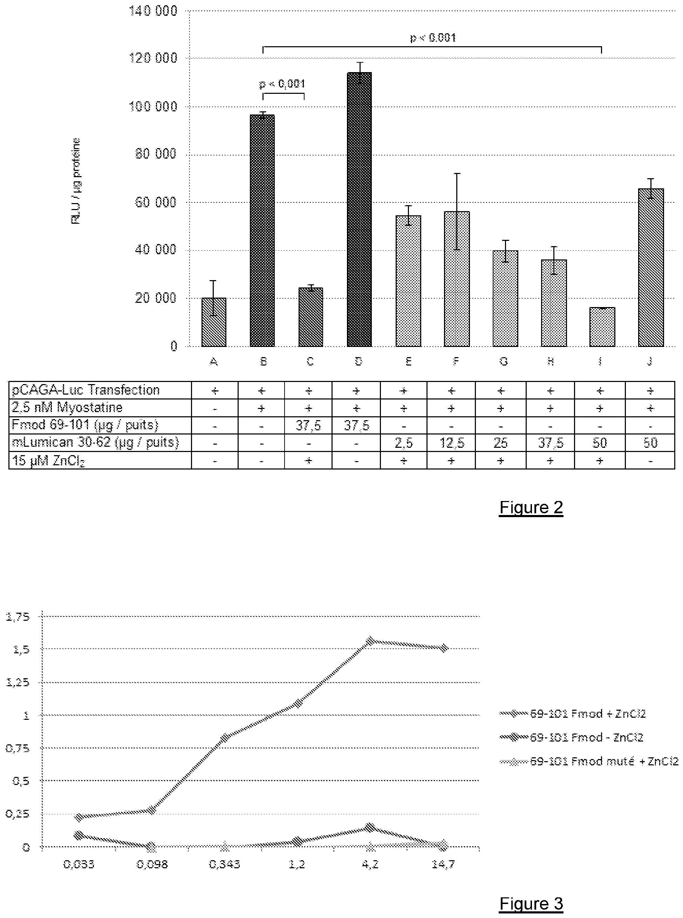

L'induction du promoteur pCAGA-Luc est mesurée selon les indications du matériel et méthode, et montrée dans les Figures 1 et 2. Une nette inhibition du promoteur, de façon dose-dépendante, est observée aussi bien avec le peptide mFmod 69-101 (Figure 1), qu'avec le peptide mLumican 30-62 (Figure 2).

### 2/ Etude in vitro de l'interaction entre la myostatine et les peptides Fibromoduline :

Comme le montre la Figure 3, plus la quantité de peptide 69-101 Fmod (SEQ ID NO : 2) est importante, plus l'absorbance est élevée. Cela indique qu'une interaction directe a lieu entre le peptide mFmod 69-101 biotinylé et la myostatine. On constate qu'un plateau est obtenu dès 4,2 µg de peptide mFmod 69-101. En revanche, aucune interaction n'est visible entre le peptide mFmod 69-101 et la myostatine en absence de Zinc. L'interaction mFmod 69-101 avec la myostatine est donc Zinc-dépendante. Par ailleurs, aucune interaction n'est visible entre le peptide mFmod biotine muté (SEQ ID NO : 3) et la myostatine, même en présence de Zinc, ce qui montre l'importance des 3 cystéines mutées en alanine.

### 3/ Etude in vivo : masse corporelle et poids des muscles et des souris à J20 après injection dans des souris mdx :

Une première série d'expériences a été réalisée sur 5 souris *mdx*, injectées par voie IM dans le Tibialis Antérieur (TA) à l'aide des formulations suivantes :
TAG : 50 µg peptide mFmod 69-101 (SEQ ID NO: 2) + 15 µM ZnCl2 dans 25µl de NaCl;
TAD : 15 µM ZnCl2 + 25 µl NaCl.

A J20, les souris ont été sacrifiées et le poids des muscles TAG et TAD a été mesuré. Les résultats sont présentés dans le tableau suivant :

| **Souris** | **Muscles** | **Poids (en mg)** | **Croissance** |
|---|---|---|---|
| **Souris 1** | **TAD 1** | 71,6 | 5,31 % |
| | **TAG 1** | 75,4 | |
| **Souris 2** | **TAD 2** | 62,2 | 19,94 % |
| | **TAG 2** | 74,6 | |
| **Souris 3** | **TAD 3** | 61,9 | 5,33 % |
| | **TAG 3** | 65,2 | |
| **Souris 4** | **TAD 4** | 65,8 | 12,31 % |
| | **TAG 4** | 73,9 | |
| **Souris 5** | **TAG 5** | 54,4 | 22,24 % |
| | **TAG 5** | 66,5 | |

Ces résultats montrent un effet sur la croissance musculaire.

### 4/ Etude comparative in vivo de l'hypertrophie des muscles 18 jours après injection dans des souris dystrophiques mdx de peptide décorine (mDCN 31-71, SEQ ID NO : 75), fibromoduline (mFmod 69-101) ou lumican (mLum 30-62):

Les muscles TAD et TAG ont été prélevés après 18 jours après l'injection des peptides, puis pesés. L'expérience a été réalisée sur cinq souris distinctes. Les résultats moyens obtenus sont présentés dans le tableau suivant :

| **Traitement** | **Hypertrophie %** |
|---|---|
| **mDCN 31-71 (SEQ ID NO: 75)** | 10,02 ± 8,3 |
| **mFmod 69-101 (SEQ ID NO : 2)** | 29,34 ± 15,76 |
| **mLum 30-62 (SEQ ID NO : 5)** | 37,26 ± 14,69 |

La différence de masse musculaire au jour 18 entre une souris *mdx* ayant ou non reçu une injection intramusculaire de décorine, fibromoduline ou lumican indique un effet plus important pour les traitements à base de peptide mFmod 69-101 et mLumican 30-62. On constate une nette augmentation de l'hypertrophie musculaire d'un facteur 2,9 et 3,7 respectivement, par rapport à la décorine.

## Revendications

1. Utilisation non thérapeutique d'une composition comprenant un fragment du lumican de moins de 100 résidus et apte à lier la myostatine ou un fragment de fibromoduline de moins de 100 résidus et apte à lier la myostatine pour augmenter la masse musculaire.

2. Utilisation selon la revendication 1, ***caractérisée* en ce que** l'augmentation de la masse musculaire vise à compenser la fonte résultant d'une immobilisation ou de la vieillesse.

3. Composition comprenant un fragment du lumican de moins de 100 résidus et apte à lier la myostatine ou un fragment de fibromoduline de moins de 100 résidus et apte à lier la myostatine pour son utilisation dans le traitement des pathologies associées à une fonte musculaire.

4. Composition pour son utilisation selon la revendication 3, ***caractérisée* en ce que** la pathologie est choisie dans le groupe comprenant : les maladies neuromusculaires, avantageusement les dystrophies musculaires telles que la myopathie de Duchenne, et les cachexies.

5. Utilisation selon la revendication 1, ***caractérisée* en ce qu'**elle est destinée à l'animal.

6. Utilisation selon l'une des revendications 1 à 2 et 5 ou composition pour son utilisation selon les revendications 3 et 4, ***caractérisée* en ce que** le fragment du lumican est un peptide comprenant la séquence CAPECNCPHSYPTAMYC (SEQ ID NO : 4) ou la séquence CAPECNCPESYPSAMYC (SEQ ID NO : 11), ou une séquence présentant au moins 50% d'identité, avantageusement 60, 70%, 80%, 90% voire 95% d'identité avec la SEQ ID NO : 4 ou 11.

7. Utilisation ou composition pour son utilisation selon la revendication 6, ***caractérisée* en ce que** le peptide comprend ou est constitué de la séquence YGQISPNCAPECNCPH SYPTAMYCDDLKLKSVP (SEQ ID NO : 5) ou la séquence YGQSSPNCAPECNCPE SYPSAMYCDELKLKSVP (SEQ ID NO : 12), ou une séquence présentant au moins 50% d'identité, avantageusement 60, 70%, 80%, 90% voire 95% d'identité avec la SEQ ID NO : 5 ou 12.

8. Utilisation selon l'une des revendications 1 à 2 et 5 ou composition pour son utilisation selon les revendications 3 et 4, ***caractérisée* en ce que** le fragment de fibromoduline apte à lier la myostatine est un peptide comprenant la séquence CPQECDCPPNFPTAMYC (SEQ ID NO : 1) ou une séquence présentant au moins 50% d'identité, avantageusement 60, 70%, 80%, 90% voire 95% d'identité avec la SEQ ID NO : 1.

9. Utilisation ou composition pour son utilisation selon la revendication 8, ***caractérisée* en ce que** le peptide comprend ou est constitué de la séquence PPPEPRDCPQECDCPPNFPTAMYCDNRNLKYLP (SEQ ID NO: 2) ou la séquence SPPDPRDCPQECDCPPNFPTAMYCDNRNLKYLP (SEQ ID NO : 10), ou une séquence présentant au moins 50% d'identité, avantageusement 60, 70%, 80%, 90% voire 95% d'identité avec la SEQ ID NO : 2 ou 10.

10. Utilisation ou composition pour son utilisation selon l'une des revendications précédentes, ***caractérisée* en ce que** la composition comprend du zinc.

11. Utilisation ou composition pour son utilisation selon l'une des revendications précédentes, ***caractérisée* en ce que** la composition est sous une forme destinée à être administrée par voie intramusculaire, intrapéritonéale, sous-cutanée, orale ou intraveineuse.

## Patentansprüche

1. Nicht therapeutische Verwendung einer Zusammensetzung mit einem Lumicanfragment von weniger als 100 Resten, das in der Lage ist, Myostatin zu binden, oder ein Fibromodulin- Fragment von weniger als 100 Resten, das in der Lage ist, Myostatin zu binden, um die Muskelmasse zu erhöhen.

2. Verwendung gemäß Anspruch 1, ***dadurch gekennzeichnet, dass*** die Erhöhung der Muskelmasse darauf abzielt, den Schwund aufgrund einer Immobilisierung oder des Alters zu kompensieren.

3. Zusammensetzung mit einem Lumicanfragment von weniger als 100 Resten, das in der Lage ist, Myostatin zu binden, oder ein Fibromodulin- Fragment von weniger als 100 Resten, das in der Lage ist, Myostatin zu binden, zur Verwendung bei der Behandlung von Pathologien in Verbindung mit Muskelschwund.

4. Zusammensetzung zur Verwendung gemäß Anspruch 3, ***dadurch gekennzeichnet, dass*** die Pathologie aus der Gruppe, die neuromuskuläre Erkrankungen, am besten Muskeldystrophien, wie die Duchenne - Myopathie, und Kachexien, umfasst, ausgewählt wird.

5. Verwendung gemäß Anspruch 1, ***dadurch gekennzeichnet, dass*** sie für Tiere bestimmt ist.

6. Verwendung gemäß einem der Ansprüche 1 bis 2 und 5 oder Zusammensetzung zur Verwendung gemäß den Ansprüchen 3 und 4, ***dadurch gekennzeichnet, dass*** es sich bei dem Lumicanfragment um ein Peptid handelt, dass die Sequenz CAPECNCPHSYPTAMYC (SEQ ID NO : 4) oder die Sequenz CAPECNCPESYPSAMYC (SEQ ID NO : 11), oder eine Sequenz, die eine mindestens 50% - ige Identität, besser noch eine 60 %- , 70%- , 80%- , 90%- oder sogar 95% - ige Identität mit der SEQ ID NO : 4 oder 11 aufweist, umfasst.

7. Verwendung oder Zusammensetzung zur Verwendung gemäß Anspruch 6, **dadurch gekennzeichnet, dass** das Peptid die Sequenz YGQISPNCAPECNCPH SYPTAMYCDDLKLKSVP (SEQ ID NO : 5) oder die Sequenz YGQSSPNCAPECNCPE SYPSAMYCDELKLKSVP (SEQ ID NO : 12), oder eine Sequenz, die eine mindestens 50% - ige Identität, besser noch eine 60 %- , 70%- , 80%- , 90%- oder sogar 95% - ige Identität mit der SEQ ID NO : 5 oder 12 aufweist, umfasst oder daraus besteht.

8. Verwendung gemäß einem der Ansprüche 1 bis 2 und 5 oder Zusammensetzung zur Verwendung gemäß den Ansprüchen 3 und 4, ***dadurch gekennzeichnet, dass*** es sich bei dem Fïbromodulinfragment das in der Lage ist, Myostatin zu binden, um ein Peptid handelt, dass die Sequenz CPQECDCPPNFPTAMYC (SEQ ID NO : 1), oder eine Sequenz, die eine mindestens 50% - ige Identität, besser noch eine 60 %- , 70%- , 80%- , 90%- oder sogar 95% - ige Identität mit der SEQ ID NO : 1 aufweist, umfasst.

9. Verwendung oder Zusammensetzung zur Verwendung gemäß Anspruch 8, ***dadurch gekennzeichnet, dass*** das Peptid die Sequenz PPPEPRDCPQECD CPPNFPTAMYCDNRNLKYLP (SEQ ID NO : 2) oder die Sequenz SPPDPRDCPQECDCPPNFPTAMYCDNRNLKYLP (SEQ ID NO : 10), oder eine Sequenz, die eine mindestens 50% - ige Identität, besser noch eine 60 %- , 70%- , 80%- , 90%- oder sogar 95% - ige Identität mit der SEQ ID NO : 2 oder 10 aufweist, umfasst oder daraus besteht.

10. Verwendung oder Zusammensetzung zur Verwendung gemäß einem der vorhergehenden Ansprüche, ***dadurch gekennzeichnet, dass*** die Zusammensetzung Zink enthält.

11. Verwendung oder Zusammensetzung zur Verwendung gemäß einem der vorhergehenden Ansprüche, ***dadurch gekennzeichnet, dass*** die Zusammensetzung eine solche Form hat, dass sie intramuskulär, intraperitoneal, subkutan, oral oder intravenös verabreicht werden kann.

## Claims

1. Non-therapeutic use of a composition comprising a fragment of lumican of less than 100 residues and capable of binding myostatin or a fragment of fibromodulin of less than 100 residues and capable of binding myostatin to increase muscle mass.

2. Use according to claim 1, ***characterized* in that** the increase in muscle mass is to compensate for wasting resulting from immobilization or old age.

3. Composition comprising a fragment of lumican of less than 100 residues and capable of binding myostatin or a fragment of fibromodulin of less than 100 residues and capable of binding myostatin for its use in the treatment of diseases associated with muscle wasting.

4. Composition for its use according to claim 3, ***characterized* in that** the disease is selected from the group consisting of: neuromuscular diseases, advantageously muscular dystrophies such as Duchenne myopathy, and cachexia.

5. Use according to claim 1, ***characterized* in that** the subject is an animal.

6. Use according to any of claims 1 to 2 and 5 or composition for its use according to claims 3 and *4,* ***characterized* in that** the fragment of lumican is a peptide comprising the sequence CAPECNCPHSYPTAMYC (SEQ ID NO: 4) or the sequence CAPECNCPESYPSAMYC (SEQ ID NO: 11), or a sequence having at least 50% identity, advantageously 60, 70%, 80%, 90% or 95% identity with SEQ ID NO: 4 or 11.

7. Use or composition for its use according to claim 6, ***characterized* in that** the peptide comprises or has the sequence YGQISPNCAPECNCPH SYPTAMYCDDLKLKSVP (SEQ ID NO: 5) or the sequence YGQSSPNCAPECNCPE SYPSAMYCDELKLKSVP (SEQ ID NO: 12), or a sequence having at least 50% identity, advantageously 60, 70%, 80%, 90% or 95% identity with the sequence SEQ ID NO: 5 or 12.

8. Use according to any of claims 1 to 2 and 5 or composition for its use according to claims 3 and *4,* ***characterized* in that** the fragment of fibromodulin capable of binding myostatin is a peptide comprising the sequence CPQECDCPPNFPTAMYC (SEQ ID NO: 1) or a sequence having at least 50% identity, advantageously 60, 70%, 80%, 90% or 95% identity with SEQ ID NO: 1.

9. Use or composition for its use according to claim 8, ***characterized* in that** the peptide comprises or has the sequence PPPEPRDCPQECDCPPNFPTAMYCDNRNLKYLP (SEQ ID NO: 2) or the sequence SPPDPRDCPQECDCPPNFPTAMYCDNRNLKYLP (SEQ ID NO: 10), or a sequence having at least 50% identity, advantageously 60, 70%, 80%, 90% or 95% identity with SEQ ID NO: 2 or 10.

10. Use or composition for its use according to any of preceding claims, ***characterized* in that** the composition includes zinc.

11. Use or composition for its use according to any of preceding claims, ***characterized* in that** the composition is in a form for administration by the intramuscular, intraperitoneal, subcutaneous, intravenous or oral path.


### SEQUENCE LISTING

<110> ASSOCIATION FRANÇAISE CONTRE LES MYOPATHIES
<120> UTILISATION DE LA FIBROMODULINE ET DU LUMICAN POUR AUGMENTER LA MASSE MUSCULAIRE
<130> A293-B-37145 PCT
<150> 1160533
   <151> 2011-11-18
<160> 75
<170> PatentIn version 3.3
<210> 1
   <211> 17
   <212> PRT
   <213> artificial sequence
<220>
   <223> 76-92 fibromoduline murine
<400> 1
   Cys Pro Gln Glu Cys Asp Cys Pro Pro Asn Phe Pro Thr Ala Met Tyr
   1               5                   10                  15
   Cys
<210> 2
   <211> 33
   <212> PRT
   <213> artificial sequence
<220>
   <223> 69-101 fibromoduline murine (Fmod)
<400> 2
   Pro Pro Pro Glu Pro Arg Asp Cys Pro Gln Glu Cys Asp Cys Pro Pro
   1               5                   10                  15
   Asn Phe Pro Thr Ala Met Tyr Cys Asp Asn Arg Asn Leu Lys Tyr Leu
               20                  25                  30
   Pro
<210> 3
   <211> 33
   <212> PRT
   <213> artificial sequence
<220>
   <223> 69-101 fibromoduline murine mutée (Fmod mutée)
<400> 3
   Pro Pro Pro Glu Pro Arg Asp Cys Pro Gln Glu Ala Asp Ala Pro Pro
   1               5                   10                  15
   Asn Phe Pro Thr Ala Met Tyr Ala Asp Asn Arg Asn Leu Lys Tyr Leu
               20                  25                  30
   Pro
<210> 4
   <211> 17
   <212> PRT
   <213> artificial sequence
<220>
   <223> 37-53 lumican murin
<400> 4
   Cys Ala Pro Glu Cys Asn Cys Pro His Ser Tyr Pro Thr Ala Met Tyr
   1               5                   10                  15
   Cys
<210> 5
   <211> 33
   <212> PRT
   <213> artificial sequence
<220>
   <223> 30-62 lumican murin
<400> 5
   Tyr Gly Gln Ile Ser Pro Asn Cys Ala Pro Glu Cys Asn Cys Pro His
   1               5                   10                  15
   Ser Tyr Pro Thr Ala Met Tyr Cys Asp Asp Leu Lys Leu Lys Ser Val
               20                  25                  30
   Pro
<210> 6
   <211> 376
   <212> PRT
   <213> Mus musculus
<400> 6
   Met Gln Trp Ala Ser Val Leu Leu Leu Ala Gly Leu Cys Ser Leu Ser
   1               5                   10                  15
   Gln Gly Gln Tyr Asp Glu Asp Ser His Trp Trp Ile Gln Tyr Leu Arg
               20                  25                  30
   Asn Gln Gln Ser Thr Tyr Tyr Asp Pro Tyr Asp Pro Tyr Pro Tyr Glu
           35                  40                  45
   Pro Ser Glu Pro Tyr Pro Tyr Gly Val Glu Glu Gly Pro Ala Tyr Ala
       50                  55                  60
   Tyr Gly Ala Pro Pro Pro Pro Glu Pro Arg Asp Cys Pro Gln Glu Cys
   65                  70                  75                  80
   Asp Cys Pro Pro Asn Phe Pro Thr Ala Met Tyr Cys Asp Asn Arg Asn
                   85                  90                  95
   Leu Lys Tyr Leu Pro Phe Val Pro Ser Arg Met Lys Tyr Val Tyr Phe
               100                 105                 110
   Gln Asn Asn Gln Ile Ser Ala Ile Gln Glu Gly Val Phe Asp Asn Ala
           115                 120                 125
   Thr Gly Leu Leu Trp Val Ala Leu His Gly Asn Gln Ile Thr Ser Asp
       130                 135                 140
   Lys Val Gly Arg Lys Val Phe Ser Lys Leu Arg His Leu Glu Arg Leu
   145                 150                 155                 160
   Tyr Leu Asp His Asn Asn Leu Thr Arg Met Pro Gly Pro Leu Pro Arg
                   165                 170                 175
   Ser Leu Arg Glu Leu His Leu Asp His Asn Gln Ile Ser Arg Val Pro
               180                 185                 190
   Asn Asn Ala Leu Glu Gly Leu Glu Asn Leu Thr Ala Leu Tyr Leu His
           195                 200                 205
   His Asn Glu Ile Gln Glu Val Gly Ser Ser Met Arg Gly Leu Arg Ser
       210                 215                 220
   Leu Ile Leu Leu Asp Leu Ser Tyr Asn His Leu Arg Arg Val Pro Asp
   225                 230                 235                 240
   Gly Leu Pro Ser Ala Leu Glu Gln Leu Tyr Leu Glu His Asn Asn Val
                   245                 250                 255
   Tyr Thr Val Pro Asp Ser Tyr Phe Arg Gly Ser Pro Lys Leu Leu Tyr
                   260                 265                 270
   Val Arg Leu Ser His Asn Ser Leu Thr Asn Asn Gly Leu Ala Thr Asn
               275                 280                 285
   Thr Phe Asn Ser Ser Ser Leu Leu Glu Leu Asp Leu Ser Tyr Asn Gln
       290                 295                 300
   Leu Gln Lys Ile Pro Pro Val Asn Thr Asn Leu Glu Asn Leu Tyr Leu
   305                 310                 315                 320
   Gln Gly Asn Arg Ile Asn Glu Phe Ser Ile Ser Ser Phe Cys Thr Val
                   325                 330                 335
   Val Asp Val Met Asn Phe Ser Lys Leu Gln Val Leu Arg Leu Asp Gly
               340                 345                 350
   Asn Glu Ile Lys Arg Ser Ala Met Pro Val Asp Ala Pro Leu Cys Leu
           355                 360                 365
   Arg Leu Ala Asn Leu Ile Glu Ile
       370                 375
<210> 7
   <211> 376
   <212> PRT
   <213> Homo sapiens
<400> 7
<210> 8
   <211> 338
   <212> PRT
   <213> Mus musculus
<400> 8
   Met Asn Val Cys Ala Phe Ser Leu Ala Leu Ala Leu Val Gly Ser Val
   1               5                   10                  15
   Ser Gly Gln Tyr Tyr Asp Tyr Asp Ile Pro Leu Phe Met Tyr Gly Gln
               20                  25                  30
   Ile Ser Pro Asn Cys Ala Pro Glu Cys Asn Cys Pro His Ser Tyr Pro
           35                  40                  45
   Thr Ala Met Tyr Cys Asp Asp Leu Lys Leu Lys Ser Val Pro Met Val
       50                  55                  60
   Pro Pro Gly Ile Lys Tyr Leu Tyr Leu Arg Asn Asn Gln Ile Asp His
   65                  70                  75                  80
   Ile Asp Glu Lys Ala Phe Glu Asn Val Thr Asp Leu Gln Trp Leu Ile
                   85                  90                  95
   Leu Asp His Asn Leu Leu Glu Asn Ser Lys Ile Lys Glu Lys Val Phe
               100                 105                 110
   Ser Lys Leu Lys Gln Leu Lys Lys Leu His Ile Asn Tyr Asn Asn Leu
           115                 120                 125
   Thr Glu Ser Val Gly Pro Leu Pro Lys Ser Leu Gln Asp Leu Gln Leu
       130                 135                 140
   Thr Asn Asn Lys Ile Ser Lys Leu Gly Ser Phe Asp Gly Leu Val Asn
   145                 150                 155                 160
   Leu Thr Phe Ile Tyr Leu Gln His Asn Gln Leu Lys Glu Asp Ala Val
                   165                 170                 175
   Ser Ala Ser Leu Lys Gly Leu Lys Ser Leu Glu Tyr Leu Asp Leu Ser
               180                 185                 190
   Phe Asn Gln Met Ser Lys Leu Pro Ala Gly Leu Pro Thr Ser Leu Leu
   195 200 205
   Thr Leu Tyr Leu Asp Asn Asn Lys Ile Ser Asn Ile Pro Asp Glu Tyr
   210 215 220
   Phe Lys Arg Phe Thr Gly Leu Gln Tyr Leu Arg Leu Ser His Asn Glu
   225 230 235 240
   Leu Ala Asp Ser Gly Val Pro Gly Asn Ser Phe Asn Ile Ser Ser Leu
   245 250 255
   Leu Glu Leu Asp Leu Ser Tyr Asn Lys Leu Lys Ser Ile Pro Thr Val
   260 265 270
   Asn Glu Asn Leu Glu Asn Tyr Tyr Leu Glu Val Asn Glu Leu Glu Lys
   275 280 285
   Phe Asp Val Lys Thr Phe Cys Lys Ile Leu Gly Pro Leu Ser Tyr Ser
   290 295 300
   Lys Ile Lys His Leu Arg Leu Asp Gly Asn Pro Leu Thr Gln Ser Ser
   305 310 315 320
   Leu Pro Pro Asp Met Tyr Glu Cys Leu Arg Val Ala Asn Glu Ile Thr
   325 330 335
   Val Asn
<210> 9
   <211> 338
   <212> PRT
   <213> Homo sapiens
<400> 9
<210> 10
   <211> 33
   <212> PRT
   <213> artificial
<220>
   <223> peptide fibromoduline humaine
<400> 10
   Ser Pro Pro Asp Pro Arg Asp Cys Pro Gln Glu Cys Asp Cys Pro Pro
   1 5 10 15
   Asn Phe Pro Thr Ala Met Tyr Cys Asp Asn Arg Asn Leu Lys Tyr Leu
   20 25 30
   Pro
<210> 11
   <211> 17
   <212> PRT
   <213> artificial sequence
<220>
   <223> 37-53 lumican humain
<400> 11
   Cys Ala Pro Glu Cys Asn Cys Pro Glu Ser Tyr Pro Ser Ala Met Tyr
   1 5 10 15
   Cys
<210> 12
   <211> 33
   <212> PRT
   <213> artificial sequence
<220>
   <223> 30-62 lumican humain
<400> 12
   Tyr Gly Gln Ser Ser Pro Asn Cys Ala Pro Glu Cys Asn Cys Pro Glu
   1 5 10 15
   Ser Tyr Pro Ser Ala Met Tyr Cys Asp Glu Leu Lys Leu Lys Ser Val
   20 25 30
   Pro
<210> 13
   <211> 373
   <212> PRT
   <213> Mus musculus
<400> 13
   Met Lys Glu Tyr Val Met Leu Leu Leu Leu Ala Val Cys Ser Ala Lys
   1 5 10 15
   Pro Phe Phe Ser Pro Ser His Thr Ala Leu Lys Asn Met Met Leu Lys
   20 25 30
   Asp Met Glu Asp Thr Asp Asp Asp Asp Asn Asp Asp Asp Asp Asn Ser
   35 40 45
   Leu Phe Pro Thr Lys Glu Pro Val Asn Pro Phe Phe Pro Phe Asp Leu
   50 55 60
   Phe Pro Thr Cys Pro Phe Gly Cys Gln Cys Tyr Ser Arg Val Val His
   65 70 75 80
   Cys Ser Asp Leu Gly Leu Thr Ser Val Pro Asn Asn Ile Pro Phe Asp
   85 90 95
   Thr Arg Met Val Asp Leu Gln Asn Asn Lys Ile Lys Glu Ile Lys Glu
   100 105 110
   Asn Asp Phe Lys Gly Leu Thr Ser Leu Tyr Ala Leu Ile Leu Asn Asn
   115 120 125
   Asn Lys Leu Thr Lys Ile His Pro Lys Thr Phe Leu Thr Thr Lys Lys
   130 135 140
   Leu Arg Arg Leu Tyr Leu Ser His Asn Gln Leu Ser Glu Ile Pro Leu
   145 150 155 160
   Asn Leu Pro Lys Ser Leu Ala Glu Leu Arg Ile His Asp Asn Lys Val
   165 170 175
   Lys Lys Ile Gln Lys Asp Thr Phe Lys Gly Met Asn Ala Leu His Val
   180 185 190
   Leu Glu Met Ser Ala Asn Pro Leu Glu Asn Asn Gly Ile Glu Pro Gly
           195                 200                 205
   Ala Phe Glu Gly Val Thr Val Phe His Ile Arg Ile Ala Glu Ala Lys
       210                 215                 220
   Leu Thr Ser Ile Pro Lys Gly Leu Pro Pro Thr Leu Leu Glu Leu His
   225                 230                 235                 240
   Leu Asp Phe Asn Lys Ile Ser Thr Val Glu Leu Glu Asp Leu Lys Arg
                   245                 250                 255
   Tyr Arg Glu Leu Gln Arg Leu Gly Leu Gly Asn Asn Arg Ile Thr Asp
               260                 265                 270
   Ile Glu Asn Gly Thr Phe Ala Asn Ile Pro Arg Val Arg Glu Ile His
           275                 280                 285
   Leu Glu His Asn Lys Leu Lys Lys Ile Pro Ser Gly Leu Gln Glu Leu
       290                 295                 300
   Lys Tyr Leu Gln Ile Ile Phe Leu His Tyr Asn Ser Ile Ala Lys Val
   305                 310                 315                 320
   Gly Val Asn Asp Phe Cys Pro Thr Val Pro Lys Met Lys Lys Ser Leu
                   325                 330                 335
   Tyr Ser Ala Ile Ser Leu Phe Asn Asn Pro Met Lys Tyr Trp Glu Ile
               340                 345                 350
   Gln Pro Ala Thr Phe Arg Cys Val Leu Gly Arg Met Ser Val Gln Leu
           355                 360                 365
   Gly Asn Val Gly Lys
       370
<210> 14
   <211> 380
   <212> PRT
   <213> Homo sapiens
<400> 14
   Met Lys Glu Tyr Val Leu Leu Leu Phe Leu Ala Leu Cys Ser Ala Lys
   1               5                   10                  15
   Pro Phe Phe Ser Pro Ser His Ile Ala Leu Lys Asn Met Met Leu Lys
               20                  25                  30
   Asp Met Glu Asp Thr Asp Asp Asp Asp Asp Asp Asp Asp Asp Asp Asp
           35                  40                  45
   Asp Asp Asp Glu Asp Asn Ser Leu Phe Pro Thr Arg Glu Pro Arg Ser
       50                  55                  60
   His Phe Phe Pro Phe Asp Leu Phe Pro Met Cys Pro Phe Gly Cys Gln
   65                  70                  75                  80
   Cys Tyr Ser Arg Val Val His Cys Ser Asp Leu Gly Leu Thr Ser Val
                   85                  90                  95
   Pro Thr Asn Ile Pro Phe Asp Thr Arg Met Leu Asp Leu Gln Asn Asn
               100                 105                 110
   Lys Ile Lys Glu Ile Lys Glu Asn Asp Phe Lys Gly Leu Thr Ser Leu
           115                 120                 125
   Tyr Gly Leu Ile Leu Asn Asn Asn Lys Leu Thr Lys Ile His Pro Lys
       130                 135                 140
   Ala Phe Leu Thr Thr Lys Lys Leu Arg Arg Leu Tyr Leu Ser His Asn
   145                 150                 155                 160
   Gln Leu Ser Glu Ile Pro Leu Asn Leu Pro Lys Ser Leu Ala Glu Leu
                   165                 170                 175
   Arg Ile His Glu Asn Lys Val Lys Lys Ile Gln Lys Asp Thr Phe Lys
               180                 185                 190
   Gly Met Asn Ala Leu His Val Leu Glu Met Ser Ala Asn Pro Leu Asp
           195                 200                 205
   Asn Asn Gly Ile Glu Pro Gly Ala Phe Glu Gly Val Thr Val Phe His
       210                 215                 220
   Ile Arg Ile Ala Glu Ala Lys Leu Thr Ser Val Pro Lys Gly Leu Pro
   225                 230                 235                 240
   Pro Thr Leu Leu Glu Leu His Leu Asp Tyr Asn Lys Ile Ser Thr Val
                   245                 250                 255
   Glu Leu Glu Asp Phe Lys Arg Tyr Lys Glu Leu Gln Arg Leu Gly Leu
               260                 265                 270
   Gly Asn Asn Lys Ile Thr Asp Ile Glu Asn Gly Ser Leu Ala Asn Ile
           275                 280                 285
   Pro Arg Val Arg Glu Ile His Leu Glu Asn Asn Lys Leu Lys Lys Ile
   290 295 300
   Pro Ser Gly Leu Pro Glu Leu Lys Tyr Leu Gln Ile Ile Phe Leu His
   305 310 315 320
   Ser Asn Ser Ile Ala Arg Val Gly Val Asn Asp Phe Cys Pro Thr Val
   325 330 335
   Pro Lys Met Lys Lys Ser Leu Tyr Ser Ala Ile Ser Leu Phe Asn Asn
   340 345 350
   Pro Val Lys Tyr Trp Glu Met Gln Pro Ala Thr Phe Arg Cys Val Leu
   355 360 365
   Ser Arg Met Ser Val Gln Leu Gly Asn Phe Gly Met
   370 375 380
<210> 15
   <211> 14
   <212> PRT
   <213> artificial sequence
<220>
   <223> fragment asporine
<400> 15
   Cys Pro Phe Gly Cys Gln Cys Tyr Ser Arg Val Val His Cys
   1 5 10
<210> 16
   <211> 36
   <212> PRT
   <213> artificial sequence
<220>
   <223> 55-90 asporine murine
<400> 16
   Pro Val Asn Pro Phe Phe Pro Phe Asp Leu Phe Pro Thr Cys Pro Phe
   1 5 10 15
   Gly Cys Gln Cys Tyr Ser Arg Val Val His Cys Ser Asp Leu Gly Leu
   20 25 30
   Thr Ser Val Pro
   35
<210> 17
   <211> 36
   <212> PRT
   <213> artificial sequence
<220>
   <223> 62-97 asporine humaine
<400> 17
   Pro Arg Ser His Phe Phe Pro Phe Asp Leu Phe Pro Met Cys Pro Phe
   1               5                   10                  15
   Gly Cys Gln Cys Tyr Ser Arg Val Val His Cys Ser Asp Leu Gly Leu
               20                  25                  30
   Thr Ser Val Pro
           35
<210> 18
   <211> 423
   <212> PRT
   <213> Mus musculus
<400> 18
   Met Gly Phe Leu Ser Pro Ile Tyr Val Leu Phe Phe Cys Phe Gly Val
   1               5                   10                  15
   Arg Val Tyr Cys Gln Tyr Glu Ala Tyr Arg Trp Asp Asp Asp Tyr Asp
               20                  25                  30
   Gln Glu Pro Asn Glu Asp Tyr Asp Pro Glu Phe Gln Phe His Gln Asn
           35                  40                  45
   Ile Glu Tyr Gly Val Pro Phe Tyr Asn Asn Ile Leu Gly Cys Ala Lys
       50                  55                  60
   Glu Cys Phe Cys Pro Thr Asn Phe Pro Thr Ser Met Tyr Cys Asp Asn
   65                  70                  75                  80
   Arg Lys Leu Lys Thr Ile Pro Ile Ile Pro Met His Ile Gln Gln Leu
                   85                  90                  95
   Asn Leu Gln Phe Asn Asp Ile Glu Ala Val Thr Ala Asn Ser Phe Ile
               100                 105                 110
   Asn Ala Thr His Leu Lys Glu Ile Asn Leu Ser His Asn Lys Ile Lys
           115                 120                 125
   Ser Gln Lys Ile Asp Tyr Gly Val Phe Ala Lys Leu Ser Asn Leu Gln
       130                 135                 140
   Gln Leu His Leu Glu His Asn Asn Leu Glu Glu Phe Pro Phe Pro Leu
   145                 150                 155                 160
   Pro Lys Ser Leu Glu Arg Leu Leu Leu Gly Tyr Asn Glu Ile Ser Ile
                   165                 170                 175
   Leu Pro Thr Asn Ala Met Asp Gly Leu Val Asn Val Thr Met Leu Asp
               180                 185                 190
   Leu Cys Tyr Asn His Leu Ser Asp Ser Met Leu Lys Glu Lys Thr Leu
           195                 200                 205
   Ser Lys Met Glu Lys Leu Met Gln Leu Asn Leu Cys Asn Asn Arg Leu
       210                 215                 220
   Glu Ser Met Pro Leu Gly Leu Pro Ser Ser Leu Met Tyr Leu Ser Leu
   225                 230                 235                 240
   Glu Asn Asn Ser Ile Ser Ser Ile Pro Asp Asn Tyr Phe Asp Lys Leu
                   245                 250                 255
   Pro Lys Leu His Ala Leu Arg Ile Ser His Asn Lys Leu Glu Asp Ile
               260                 265                 270
   Pro Tyr Asp Ile Phe Asn Leu Ser Asn Leu Ile Glu Leu Asn Val Gly
           275                 280                 285
   His Asn Lys Leu Lys Gln Ala Phe Tyr Ile Pro Arg Asn Leu Glu His
       290                 295                 300
   Leu Tyr Leu Gln Asn Asn Glu Ile Glu Ser Ile Asn Val Thr Met Ile
   305                 310                 315                 320
   Cys Pro Ser Pro Asp Pro Val His His His His Leu Thr Tyr Leu Arg
                   325                 330                 335
   Val Asp Gln Asn Lys Leu Lys Glu Pro Ile Ser Ser Tyr Ile Phe Phe
               340                 345                 350
   Cys Phe Pro Arg Ile His Ser Ile Tyr Tyr Gly Glu Gln Arg Ser Thr
           355                 360                 365
   Asn Gly Glu Thr Ile Gln Leu Lys Thr Gln Val Phe Arg Ser Tyr Gln
       370                 375                 380
   Glu Glu Glu Glu Glu Asp Asp His Asp Ser Gln Asp Asn Thr Leu Glu
   385                 390                 395                 400
   Gly Gln Glu Val Ser Asp Glu His Tyr Asn Ser His Tyr Tyr Glu Met
                   405                 410                 415
   Gln Glu Trp Gln Asp Thr Ile
               420
<210> 19
   <211> 421
   <212> PRT
   <213> Homo sapiens
<400> 19
   Met Gly Phe Leu Ser Pro Ile Tyr Val Ile Phe Phe Phe Phe Gly Val
   1               5                   10                  15
   Lys Val His Cys Gln Tyr Glu Thr Tyr Gln Trp Asp Glu Asp Tyr Asp
               20                  25                  30
   Gln Glu Pro Asp Asp Asp Tyr Gln Thr Gly Phe Pro Phe Arg Gln Asn
           35                  40                  45
   Val Asp Tyr Gly Val Pro Phe His Gln Tyr Thr Leu Gly Cys Val Ser
       50                  55                  60
   Glu Cys Phe Cys Pro Thr Asn Phe Pro Ser Ser Met Tyr Cys Asp Asn
   65                  70                  75                  80
   Arg Lys Leu Lys Thr Ile Pro Asn Ile Pro Met His Ile Gln Gln Leu
                   85                  90                  95
   Tyr Leu Gln Phe Asn Glu Ile Glu Ala Val Thr Ala Asn Ser Phe Ile
               100                 105                 110
   Asn Ala Thr His Leu Lys Glu Ile Asn Leu Ser His Asn Lys Ile Lys
           115                 120                 125
   Ser Gln Lys Ile Asp Tyr Gly Val Phe Ala Lys Leu Pro Asn Leu Leu
       130                 135                 140
   Gln Leu His Leu Glu His Asn Asn Leu Glu Glu Phe Pro Phe Pro Leu
   145                 150                 155                 160
   Pro Lys Ser Leu Glu Arg Leu Leu Leu Gly Tyr Asn Glu Ile Ser Lys
                   165                 170                 175
   Leu Gln Thr Asn Ala Met Asp Gly Leu Val Asn Leu Thr Met Leu Asp
               180                 185                 190
   Leu Cys Tyr Asn Tyr Leu His Asp Ser Leu Leu Lys Asp Lys Ile Phe
   195 200 205
   Ala Lys Met Glu Lys Leu Met Gln Leu Asn Leu Cys Ser Asn Arg Leu
   210 215 220
   Glu Ser Met Pro Pro Gly Leu Pro Ser Ser Leu Met Tyr Leu Ser Leu
   225 230 235 240
   Glu Asn Asn Ser Ile Ser Ser Ile Pro Glu Lys Tyr Phe Asp Lys Leu
   245 250 255
   Pro Lys Leu His Thr Leu Arg Met Ser His Asn Lys Leu Gln Asp Ile
   260 265 270
   Pro Tyr Asn Ile Phe Asn Leu Pro Asn Ile Val Glu Leu Ser Val Gly
   275 280 285
   His Asn Lys Leu Lys Gln Ala Phe Tyr Ile Pro Arg Asn Leu Glu His
   290 295 300
   Leu Tyr Leu Gln Asn Asn Glu Ile Glu Lys Met Asn Leu Thr Val Met
   305 310 315 320
   Cys Pro Ser Ile Asp Pro Leu His Tyr His His Leu Thr Tyr Ile Arg
   325 330 335
   Val Asp Gln Asn Lys Leu Lys Glu Pro Ile Ser Ser Tyr Ile Phe Phe
   340 345 350
   Cys Phe Pro His Ile His Thr Ile Tyr Tyr Gly Glu Gln Arg Ser Thr
   355 360 365
   Asn Gly Gln Thr Ile Gln Leu Lys Thr Gln Val Phe Arg Arg Phe Pro
   370 375 380
   Asp Asp Asp Asp Glu Ser Glu Asp His Asp Asp Pro Asp Asn Ala His
   385 390 395 400
   Glu Ser Pro Glu Gln Glu Gly Ala Glu Gly His Phe Asp Leu His Tyr
   405 410 415
   Tyr Glu Asn Gln Glu
   420
<210> 20
   <211> 17
   <212> PRT
   <213> artificial sequence
<220>
   <223> fragment osteoadherine murine
<400> 20
   Cys Ala Lys Glu Cys Phe Cys Pro Thr Asn Phe Pro Thr Ser Met Tyr
   1               5                   10                  15
   Cys
<210> 21
   <211> 17
   <212> PRT
   <213> artificial sequence
<220>
   <223> fragment osteoadherine humaine
<400> 21
   Cys Val Ser Glu Cys Phe Cys Pro Thr Asn Phe Pro Ser Ser Met Tyr
   1               5                   10                  15
   Cys
<210> 22
   <211> 37
   <212> PRT
   <213> artificial sequence
<220>
   <223> 51-87 osteoadherine murine
<400> 22
   Tyr Gly Val Pro Phe Tyr Asn Asn Ile Leu Gly Cys Ala Lys Glu Cys
   1               5                   10                  15
   Phe Cys Pro Thr Asn Phe Pro Thr Ser Met Tyr Cys Asp Asn Arg Lys
               20                  25                  30
   Leu Lys Thr Ile Pro
           35
<210> 23
   <211> 37
   <212> PRT
   <213> artificial sequence
<220>
<210> 24
   <211> 378
   <212> PRT
   <213> Mus musculus
<400> 24
   Met Arg Ala Ser Phe Phe Trp Leu Leu Pro Leu Leu Leu Ile Leu Ala
   1 5 10 15
   Ser Val Ala Gln Gly Gln Pro Thr Arg Pro Lys Pro Gly Ile Arg Arg
   20 25 30
   Lys Pro Lys Pro Arg Pro Thr Pro Arg Phe Pro Gln Ala Pro Glu Pro
   35 40 45
   Ala Glu Pro Thr Asp Leu Pro Pro Pro Leu Pro Pro Gly Pro Pro Ser
   50 55 60
   Val Phe Pro Asp Cys Pro Arg Glu Cys Tyr Cys Pro Pro Asp Phe Pro
   65 70 75 80
   Ser Ala Leu Tyr Cys Asp Ser Arg Asn Leu Arg Arg Val Pro Val Ile
   85 90 95
   Pro Pro Arg Ile His Tyr Leu Tyr Leu Gln Asn Asn Phe Ile Thr Glu
   100 105 110
   Leu Pro Leu Glu Ser Phe Gln Asn Ala Thr Gly Leu Arg Trp Val Asn
   115 120 125
   Leu Asp Asn Asn Arg Ile Arg Lys Val Asp Gln Arg Val Leu Gly Lys
   130 135 140
   Leu Pro Ser Leu Ala Phe Leu Tyr Met Glu Lys Asn Gln Leu Glu Glu
   145 150 155 160
   Val Pro Ser Ala Leu Pro Arg Asn Leu Glu Gln Leu Arg Leu Ser Gln
                   165                 170                 175
   Asn Leu Ile Ser Arg Ile Pro Pro Gly Val Phe Ser Lys Leu Glu Asn
               180                 185                 190
   Leu Leu Leu Leu Asp Leu Gln His Asn Arg Leu Ser Asp Gly Val Phe
           195                 200                 205
   Lys Ala Asp Thr Phe Gln Gly Leu Lys Asn Leu Met Gln Leu Asn Leu
       210                 215                 220
   Ala His Asn Ile Leu Arg Lys Met Pro Pro Lys Val Pro Gln Ala Ile
   225                 230                 235                 240
   His Gln Leu Tyr Leu Asp Ser Asn Lys Ile Glu Thr Ile Pro Asn Gly
                   245                 250                 255
   Tyr Phe Lys Asp Phe Pro Asn Leu Ala Phe Ile Arg Met Asn Tyr Asn
               260                 265                 270
   Lys Leu Ser Asp Arg Gly Leu Pro Lys Asn Ser Phe Asn Ile Ser Asn
           275                 280                 285
   Leu Leu Val Leu His Leu Ser His Asn Lys Ile Ser Asn Val Pro Ala
       290                 295                 300
   Ile Ser Asn Lys Leu Glu His Leu Tyr Leu Asn Asn Asn Ser Ile Glu
   305                 310                 315                 320
   Lys Ile Asn Gly Thr Gln Ile Cys Pro Asn Asn Leu Val Ala Phe His
                   325                 330                 335
   Asp Phe Ser Ser Asp Leu Glu Asn Val Pro His Leu Arg Tyr Leu Arg
               340                 345                 350
   Leu Asp Gly Asn Phe Leu Lys Pro Pro Ile Pro Leu Asp Leu Met Met
           355                 360                 365
   Cys Phe Arg Leu Leu Gln Ser Val Val Ile
       370                 375
<210> 25
   <211> 382
   <212> PRT
   <213> Homo sapiens
<400> 25
   Met Arg Ser Pro Leu Cys Trp Leu Leu Pro Leu Leu Ile Leu Ala Ser
   1               5                   10                  15
   Val Ala Gln Gly Gln Pro Thr Arg Arg Pro Arg Pro Gly Thr Gly Pro
               20                  25                  30
   Gly Arg Arg Pro Arg Pro Arg Pro Arg Pro Thr Pro Ser Phe Pro Gln
           35                  40                  45
   Pro Asp Glu Pro Ala Glu Pro Thr Asp Leu Pro Pro Pro Leu Pro Pro
       50                  55                  60
   Gly Pro Pro Ser Ile Phe Pro Asp Cys Pro Arg Glu Cys Tyr Cys Pro
   65                  70                  75                  80
   Pro Asp Phe Pro Ser Ala Leu Tyr Cys Asp Ser Arg Asn Leu Arg Lys
                   85                  90                  95
   Val Pro Val Ile Pro Pro Arg Ile His Tyr Leu Tyr Leu Gln Asn Asn
               100                 105                 110
   Phe Ile Thr Glu Leu Pro Val Glu Ser Phe Gln Asn Ala Thr Gly Leu
           115                 120                 125
   Arg Trp Ile Asn Leu Asp Asn Asn Arg Ile Arg Lys Ile Asp Gln Arg
       130                 135                 140
   Val Leu Glu Lys Leu Pro Gly Leu Val Phe Leu Tyr Met Glu Lys Asn
   145                 150                 155                 160
   Gln Leu Glu Glu Val Pro Ser Ala Leu Pro Arg Asn Leu Glu Gln Leu
                   165                 170                 175
   Arg Leu Ser Gln Asn His Ile Ser Arg Ile Pro Pro Gly Val Phe Ser
               180                 185                 190
   Lys Leu Glu Asn Leu Leu Leu Leu Asp Leu Gln His Asn Arg Leu Ser
           195                 200                 205
   Asp Gly Val Phe Lys Pro Asp Thr Phe His Gly Leu Lys Asn Leu Met
       210                 215                 220
   Gln Leu Asn Leu Ala His Asn Ile Leu Arg Lys Met Pro Pro Arg Val
   225                 230                 235                 240
   Pro Thr Ala Ile His Gln Leu Tyr Leu Asp Ser Asn Lys Ile Glu Thr
                   245                 250                 255
   Ile Pro Asn Gly Tyr Phe Lys Ser Phe Pro Asn Leu Ala Phe Ile Arg
   260 265 270
   Leu Asn Tyr Asn Lys Leu Thr Asp Arg Gly Leu Pro Lys Asn Ser Phe
   275 280 285
   Asn Ile Ser Asn Leu Leu Val Leu His Leu Ser His Asn Arg Ile Ser
   290 295 300
   Ser Val Pro Ala Ile Asn Asn Arg Leu Glu His Leu Tyr Leu Asn Asn
   305 310 315 320
   Asn Ser Ile Glu Lys Ile Asn Gly Thr Gln Ile Cys Pro Asn Asp Leu
   325 330 335
   Val Ala Phe His Asp Phe Ser Ser Asp Leu Glu Asn Val Pro His Leu
   340 345 350
   Arg Tyr Leu Arg Leu Asp Gly Asn Tyr Leu Lys Pro Pro Ile Pro Leu
   355 360 365
   Asp Leu Met Met Cys Phe Arg Leu Leu Gln Ser Val Val Ile
   370 375 380
<210> 26
   <211> 13
   <212> PRT
   <213> artificial sequence
<220>
   <223> fragment PRELP
<400> 26
   Cys Tyr Cys Pro Pro Asp Phe Pro Ser Ala Leu Tyr Cys
   1 5 10
<210> 27
   <211> 33
   <212> PRT
   <213> artificial sequence
<220>
   <223> 62-94 PRELP murin
<400> 27
   Pro Pro Ser Val Phe Pro Asp Cys Pro Arg Glu Cys Tyr Cys Pro Pro
   1               5                   10                  15
   Asp Phe Pro Ser Ala Leu Tyr Cys Asp Ser Arg Asn Leu Arg Arg Val
               20                  25                  30
   Pro
<210> 28
   <211> 33
   <212> PRT
   <213> artificial sequence
<220>
   <223> 66-98 PRELP humain
<400> 28
   Pro Pro Ser Ile Phe Pro Asp Cys Pro Arg Glu Cys Tyr Cys Pro Pro
   1               5                   10                  15
   Asp Phe Pro Ser Ala Leu Tyr Cys Asp Ser Arg Asn Leu Arg Lys Val
               20                  25                  30
   Pro
<210> 29
   <211> 322
   <212> PRT
   <213> Mus musculus
<400> 29
   Met Gly Met Leu Ala Arg Val Ala Leu Gly Leu Ile Ile Ile Asp Ala
   1               5                   10                  15
   Val Leu Ala Ala Pro Thr Thr Glu Leu Phe Asn Tyr Asp Ser Glu Val
               20                  25                  30
   Tyr Asp Ala Ile Leu Glu Asp Thr Gly Thr Phe Tyr Asn Tyr Glu His
           35                  40                  45
   Ile Pro Asp Asn His Val Glu Asn Glu Lys Val Ser Glu Arg Leu Ser
       50                  55                  60
   Gly Asn Arg Glu Leu Leu Thr Pro Gly Pro Gln Leu Gly Asp Asn Gln
   65                  70                  75                  80
   Asp Glu Asp Lys Asp Glu Glu Ser Thr Pro Arg Leu Ile Asp Gly Ser
                   85                  90                  95
   Ser Pro Gln Glu Pro Glu Phe Pro Gly Leu Leu Gly Pro His Thr Asn
               100                 105                 110
   Glu Asp Phe Pro Thr Cys Leu Leu Cys Thr Cys Ile Ser Thr Thr Val
   115 120 125
   Tyr Cys Asp Asp His Glu Leu Asp Ala Ile Pro Pro Leu Pro Lys Lys
   130 135 140
   Thr Thr Tyr Phe Tyr Ser Arg Phe Asn Arg Ile Lys Lys Ile Asn Lys
   145 150 155 160
   Asn Asp Phe Ala Ser Leu Asn Asp Leu Lys Arg Ile Asp Leu Thr Ser
   165 170 175
   Asn Leu Ile Ser Glu Ile Asp Glu Asp Ala Phe Arg Lys Leu Pro His
   180 185 190
   Leu Gln Glu Leu Val Leu Arg Asp Asn Lys Ile Lys Gln Leu Pro Glu
   195 200 205
   Leu Pro Asn Thr Leu Thr Phe Ile Asp Ile Ser Asn Asn Arg Leu Gly
   210 215 220
   Arg Lys Gly Ile Lys Gln Glu Ala Phe Lys Asp Met Tyr Asp Leu His
   225 230 235 240
   His Leu Tyr Ile Thr Asp Asn Ser Leu Asp His Ile Pro Leu Pro Leu
   245 250 255
   Pro Glu Ser Leu Arg Ala Leu His Leu Gln Asn Asn Asp Ile Leu Glu
   260 265 270
   Met His Glu Asp Thr Phe Cys Asn Val Lys Asn Leu Thr Tyr Val Arg
   275 280 285
   Lys Ala Leu Glu Asp Ile Arg Leu Asp Gly Asn Pro Ile Asn Leu Ser
   290 295 300
   Arg Thr Pro Gln Ala Tyr Met Cys Leu Pro Arg Leu Pro Ile Gly Ser
   305 310 315 320
   Phe Ile
<210> 30
   <211> 322
   <212> PRT
   <213> Homo sapiens
<400> 30
   Met Lys Thr Leu Ala Gly Leu Val Leu Gly Leu Val Ile Phe Asp Ala
   1 5 10 15
   Ala Val Thr Ala Pro Thr Leu Glu Ser Ile Asn Tyr Asp Ser Glu Thr
   20 25 30
   Tyr Asp Ala Thr Leu Glu Asp Leu Asp Asn Leu Tyr Asn Tyr Glu Asn
   35 40 45
   Ile Pro Val Gly Lys Val Glu Ile Glu Ile Ala Thr Val Met Pro Ser
   50 55 60
   Gly Asn Arg Glu Leu Leu Thr Pro Pro Pro Gln Pro Glu Lys Ala Gln
   65 70 75 80
   Glu Glu Glu Glu Glu Glu Glu Ser Thr Pro Arg Leu Ile Asp Gly Ser
   85 90 95
   Ser Pro Gln Glu Pro Glu Phe Thr Gly Val Leu Gly Pro His Thr Asn
   100 105 110
   Glu Asp Phe Pro Thr Cys Leu Leu Cys Thr Cys Ile Ser Thr Thr Val
   115 120 125
   Tyr Cys Asp Asp His Glu Leu Asp Ala Ile Pro Pro Leu Pro Lys Asn
   130 135 140
   Thr Ala Tyr Phe Tyr Ser Arg Phe Asn Arg Ile Lys Lys Ile Asn Lys
   145 150 155 160
   Asn Asp Phe Ala Ser Leu Ser Asp Leu Lys Arg Ile Asp Leu Thr Ser
   165 170 175
   Asn Leu Ile Ser Glu Ile Asp Glu Asp Ala Phe Arg Lys Leu Pro Gln
   180 185 190
   Leu Arg Glu Leu Val Leu Arg Asp Asn Lys Ile Arg Gln Leu Pro Glu
   195 200 205
   Leu Pro Thr Thr Leu Thr Phe Ile Asp Ile Ser Asn Asn Arg Leu Gly
   210 215 220
   Arg Lys Gly Ile Lys Gln Glu Ala Phe Lys Asp Met Tyr Asp Leu His
   225 230 235 240
   His Leu Tyr Leu Thr Asp Asn Asn Leu Asp His Ile Pro Leu Pro Leu
   245 250 255
   Pro Glu Asn Leu Arg Ala Leu His Leu Gln Asn Asn Asn Ile Leu Glu
   260 265 270
   Met His Glu Asp Thr Phe Cys Asn Val Lys Asn Leu Thr Tyr Ile Arg
   275 280 285
   Lys Ala Leu Glu Asp Ile Arg Leu Asp Gly Asn Pro Ile Asn Leu Ser
   290 295 300
   Lys Thr Pro Gln Ala Tyr Met Cys Leu Pro Arg Leu Pro Val Gly Ser
   305 310 315 320
   Leu Val
<210> 31
   <211> 13
   <212> PRT
   <213> artificial sequence
<220>
   <223> fragment epiphycan
<400> 31
   Cys Leu Leu Cys Thr Cys Ile Ser Thr Thr Val Tyr Cys
   1 5 10
<210> 32
   <211> 29
   <212> PRT
   <213> artificial sequence
<220>
   <223> 111-139 epiphycan
<400> 32
   Thr Asn Glu Asp Phe Pro Thr Cys Leu Leu Cys Thr Cys Ile Ser Thr
   1 5 10 15
   Thr Val Tyr Cys Asp Asp His Glu Leu Asp Ala Ile Pro
   20 25
<210> 33
   <211> 298
   <212> PRT
   <213> Mus musculus
<400> 33
   Met Glu Thr Val His Ser Thr Phe Leu Leu Leu Leu Phe Val Pro Leu
   1 5 10 15
   Thr Gln Gln Ala Pro Gln Ser Gln Leu Asp Ser His Val Asn Tyr Glu
               20                  25                  30
   Tyr Ala Thr Gly Asn Ser Glu Glu Thr Lys Phe Ser Gln Asp Tyr Glu
           35                  40                  45
   Asp Lys Tyr Leu Asp Gly Lys Ser Ile Lys Glu Lys Glu Thr Met Ile
       50                  55                  60
   Ile Pro Asp Glu Lys Ser Leu Gln Leu Gln Lys Asp Glu Val Ile Pro
   65                  70                  75                  80
   Ser Leu Pro Thr Lys Lys Glu Asn Asp Glu Met Pro Thr Cys Leu Leu
                   85                  90                  95
   Cys Val Cys Leu Ser Gly Ser Val Tyr Cys Glu Glu Val Asp Ile Asp
               100                 105                 110
   Ala Val Pro Pro Leu Pro Lys Glu Ser Ala Tyr Leu Tyr Ala Arg Phe
           115                 120                 125
   Asn Lys Ile Lys Lys Leu Thr Ala Lys Asp Phe Ala Asp Met Pro Asn
       130                 135                 140
   Leu Arg Arg Leu Asp Phe Thr Gly Asn Leu Ile Glu Asp Ile Glu Asp
   145                 150                 155                 160
   Gly Thr Phe Ser Lys Leu Ser Leu Leu Glu Glu Leu Thr Leu Ala Glu
                   165                 170                 175
   Asn Gln Leu Leu Arg Leu Pro Val Leu Pro Pro Lys Leu Thr Leu Leu
               180                 185                 190
   Asn Ala Lys His Asn Lys Ile Lys Ser Lys Gly Ile Lys Ala Asn Thr
           195                 200                 205
   Phe Lys Lys Leu Asn Lys Leu Ser Phe Leu Tyr Leu Asp His Asn Asp
       210                 215                 220
   Leu Glu Ser Val Pro Pro Asn Leu Pro Glu Ser Leu Arg Val Ile His
   225                 230                 235                 240
   Leu Gln Phe Asn Ser Ile Ser Ser Leu Thr Asp Asp Thr Phe Cys Lys
                   245                 250                 255
   Ala Asn Asp Thr Arg Tyr Ile Arg Glu Arg Ile Glu Glu Ile Arg Leu
               260                 265                 270
   Glu Gly Asn Pro Ile Ala Leu Gly Lys His Pro Asn Ser Phe Ile Cys
           275                 280                 285
   Leu Lys Arg Leu Pro Ile Gly Ser Tyr Phe
       290                 295
<210> 34
   <211> 298
   <212> PRT
   <213> Homo sapiens
<400> 34
   Met Lys Thr Leu Gln Ser Thr Leu Leu Leu Leu Leu Leu Val Pro Leu
   1               5                   10                  15
   Ile Lys Pro Ala Pro Pro Thr Gln Gln Asp Ser Arg Ile Ile Tyr Asp
               20                  25                  30
   Tyr Gly Thr Asp Asn Phe Glu Glu Ser Ile Phe Ser Gln Asp Tyr Glu
           35                  40                  45
   Asp Lys Tyr Leu Asp Gly Lys Asn Ile Lys Glu Lys Glu Thr Val Ile
       50                  55                  60
   Ile Pro Asn Glu Lys Ser Leu Gln Leu Gln Lys Asp Glu Ala Ile Thr
   65                  70                  75                  80
   Pro Leu Pro Pro Lys Lys Glu Asn Asp Glu Met Pro Thr Cys Leu Leu
                   85                  90                  95
   Cys Val Cys Leu Ser Gly Ser Val Tyr Cys Glu Glu Val Asp Ile Asp
               100                 105                 110
   Ala Val Pro Pro Leu Pro Lys Glu Ser Ala Tyr Leu Tyr Ala Arg Phe
           115                 120                 125
   Asn Lys Ile Lys Lys Leu Thr Ala Lys Asp Phe Ala Asp Ile Pro Asn
       130                 135                 140
   Leu Arg Arg Leu Asp Phe Thr Gly Asn Leu Ile Glu Asp Ile Glu Asp
   145                 150                 155                 160
   Gly Thr Phe Ser Lys Leu Ser Leu Leu Glu Glu Leu Ser Leu Ala Glu
                   165                 170                 175
   Asn Gln Leu Leu Lys Leu Pro Val Leu Pro Pro Lys Leu Thr Leu Phe
               180                 185                 190
   Asn Ala Lys Tyr Asn Lys Ile Lys Ser Arg Gly Ile Lys Ala Asn Ala
   195 200 205
   Phe Lys Lys Leu Asn Asn Leu Thr Phe Leu Tyr Leu Asp His Asn Ala
   210 215 220
   Leu Glu Ser Val Pro Leu Asn Leu Pro Glu Ser Leu Arg Val Ile His
   225 230 235 240
   Leu Gln Phe Asn Asn Ile Ala Ser Ile Thr Asp Asp Thr Phe Cys Lys
   245 250 255
   Ala Asn Asp Thr Ser Tyr Ile Arg Asp Arg Ile Glu Glu Ile Arg Leu
   260 265 270
   Glu Gly Asn Pro Ile Val Leu Gly Lys His Pro Asn Ser Phe Ile Cys
   275 280 285
   Leu Lys Arg Leu Pro Ile Gly Ser Tyr Phe
   290 295
<210> 35
   <211> 13
   <212> PRT
   <213> artificial sequence
<220>
   <223> fragment osteoglycine
<400> 35
   Cys Leu Leu Cys Val Cys Leu Ser Gly Ser Val Tyr Cys
   1 5 10
<210> 36
   <211> 30
   <212> PRT
   <213> artificial sequence
<220>
   <223> 86-115 osteoglycine
<400> 36
   Lys Glu Asn Asp Glu Met Pro Thr Cys Leu Leu Cys Val Cys Leu Ser
   1 5 10 15
   Gly Ser Val Tyr Cys Glu Glu Val Asp Ile Asp Ala Val Pro
   20 25 30
<210> 37
   <211> 328
   <212> PRT
   <213> Mus musculus
<400> 37
   Met Lys Phe Leu Ala Phe Leu Ser Leu Leu Ser Leu Val Leu Gln Lys
   1               5                   10                  15
   Ala Glu Thr Ala Ser Leu Leu Gly Glu Arg Glu Arg Glu Glu Gln Ser
               20                  25                  30
   Pro Glu Glu Gly Asp Thr Tyr Ala Ser Leu Tyr Val Gly Asn His Thr
           35                  40                  45
   Leu Ser Ile Glu Asp Tyr Asn Glu Val Ile Asp Leu Ser Asn Tyr Glu
       50                  55                  60
   Glu Leu Ala Asp Tyr Gly Asp Gln Ile Pro Glu Ala Lys Ile Ser Asn
   65                  70                  75                  80
   Leu Thr Leu Pro Thr Arg Thr Ser Pro Thr Ser Thr Val Ala Gln Lys
                   85                  90                  95
   Thr Leu Ser Pro Asn Leu Thr Met Ala Val Pro Thr Thr Thr Gly Leu
               100                 105                 110
   Leu Asn Ser Gln Ser Ser His Gly Leu Pro Thr Cys Leu Val Cys Val
           115                 120                 125
   Cys Leu Gly Ser Ser Val Tyr Cys Asp Asp Ala Asp Leu Glu Asn Ile
       130                 135                 140
   Pro Pro Leu Pro Gln Met Thr Thr Tyr Leu Tyr Ala Arg Phe Asn His
   145                 150                 155                 160
   Ile Ser His Ile Gln Ala Gly Asp Phe Lys Gly Leu Thr Lys Leu Arg
                   165                 170                 175
   Arg Ile Asp Leu Ser Gly Asn Ser Ile Ser Ser Ile His Asn Asp Ala
               180                 185                 190
   Leu Arg Leu Leu Pro Ala Leu Gln Asp Leu Ile Leu Pro Glu Asn Gln
           195                 200                 205
   Leu Ala Ala Leu Pro Val Leu Pro Ser Gly Ile Glu Phe Leu Asp Val
       210                 215                 220
   Arg Leu Asn Arg Leu Gln Ser Ser Gly Ile Gln Pro Glu Ala Phe Val
   225                 230                 235                 240
   Ala Leu Lys Lys Leu Gln Phe Leu Tyr Leu Ala Asn Asn Met Leu Asp
                   245                 250                 255
   Ser Ile Pro Gly Pro Leu Pro Leu Ser Leu Arg Ser Leu His Leu Gln
               260                 265                 270
   Asn Asn Met Ile Glu Thr Met Glu Ser Asp Thr Phe Cys Asp Thr Gly
           275                 280                 285
   Glu His Arg His Glu Arg Arg Gln Leu Glu Asp Ile Arg Leu Asp Gly
       290                 295                 300
   Asn Pro Ile Asn Leu Ser Leu Phe Pro Glu Ala Tyr Phe Cys Leu Pro
   305                 310                 315                 320
   Arg Leu Pro Val Gly His Phe Thr
                   325
<210> 38
   <211> 332
   <212> PRT
   <213> Homo sapiens
<400> 38
   Met Arg Leu Leu Ala Phe Leu Ser Leu Leu Ala Leu Val Leu Gln Glu
   1               5                   10                  15
   Thr Gly Thr Ala Ser Leu Pro Arg Lys Glu Arg Lys Arg Arg Glu Glu
               20                  25                  30
   Gln Met Pro Arg Glu Gly Asp Ser Phe Glu Val Leu Pro Leu Arg Asn
           35                  40                  45
   Asp Val Leu Asn Pro Asp Asn Tyr Gly Glu Val Ile Asp Leu Ser Asn
       50                  55                  60
   Tyr Glu Glu Leu Thr Asp Tyr Gly Asp Gln Leu Pro Glu Val Lys Val
   65                  70                  75                  80
   Thr Ser Leu Ala Pro Ala Thr Ser Ile Ser Pro Ala Lys Ser Thr Thr
                   85                  90                  95
   Ala Pro Gly Thr Pro Ser Ser Asn Pro Thr Met Thr Arg Pro Thr Thr
               100                 105                 110
   Ala Gly Leu Leu Leu Ser Ser Gln Pro Asn His Gly Leu Pro Thr Cys
                   115                 120                 125
   Leu Val Cys Val Cys Leu Gly Ser Ser Val Tyr Cys Asp Asp Ile Asp
       130                 135                 140
   Leu Glu Asp Ile Pro Pro Leu Pro Arg Arg Thr Ala Tyr Leu Tyr Ala
   145                 150                 155                 160
   Arg Phe Asn Arg Ile Ser Arg Ile Arg Ala Glu Asp Phe Lys Gly Leu
                   165                 170                 175
   Thr Lys Leu Lys Arg Ile Asp Leu Ser Asn Asn Leu Ile Ser Ser Ile
               180                 185                 190
   Asp Asn Asp Ala Phe Arg Leu Leu His Ala Leu Gln Asp Leu Ile Leu
           195                 200                 205
   Pro Glu Asn Gln Leu Glu Ala Leu Pro Val Leu Pro Ser Gly Ile Glu
       210                 215                 220
   Phe Leu Asp Val Arg Leu Asn Arg Leu Gln Ser Ser Gly Ile Gln Pro
   225                 230                 235                 240
   Ala Ala Phe Arg Ala Met Glu Lys Leu Gln Phe Leu Tyr Leu Ser Asp
                   245                 250                 255
   Asn Leu Leu Asp Ser Ile Pro Gly Pro Leu Pro Leu Ser Leu Arg Ser
               260                 265                 270
   Val His Leu Gln Asn Asn Leu Ile Glu Thr Met Gln Arg Asp Val Phe
           275                 280                 285
   Cys Asp Pro Glu Glu His Lys His Thr Arg Arg Gln Leu Glu Asp Ile
       290                 295                 300
   Arg Leu Asp Gly Asn Pro Ile Asn Leu Ser Leu Phe Pro Ser Ala Tyr
   305                 310                 315                 320
   Phe Cys Leu Pro Arg Leu Pro Ile Gly Arg Phe Thr
                   325                 330
<210> 39
   <211> 13
   <212> PRT
   <213> artificial sequence
<220>
   <223> fragment opticine
<400> 39
   Cys Leu Val Cys Val Cys Leu Gly Ser Ser Val Tyr Cys
   1               5                   10
<210> 40
   <211> 32
   <212> PRT
   <213> artificial sequence
<220>
   <223> 114-145 opticine murine
<400> 40
   Asn Ser Gln Ser Ser His Gly Leu Pro Thr Cys Leu Val Cys Val Cys
   1               5                   10                  15
   Leu Gly Ser Ser Val Tyr Cys Asp Asp Ala Asp Leu Glu Asn Ile Pro
               20                  25                  30
<210> 41
   <211> 32
   <212> PRT
   <213> artificial sequence
<220>
   <223> 118-149 opticine humaine
<400> 41
   Ser Ser Gln Pro Asn His Gly Leu Pro Thr Cys Leu Val Cys Val Cys
   1               5                   10                  15
   Leu Gly Ser Ser Val Tyr Cys Asp Asp Ile Asp Leu Glu Asp Ile Pro
               20                  25                  30
<210> 42
   <211> 358
   <212> PRT
   <213> Mus musculus
<400> 42
   Met Ala Arg Ala Leu Leu Phe Ser Leu Val Phe Leu Ala Ile Leu Leu
   1               5                   10                  15
   Pro Ala Leu Ala Ala Cys Pro Gln Asn Cys His Cys His Gly Asp Leu
               20                  25                  30
   Gln His Val Ile Cys Asp Lys Val Gly Leu Gln Lys Ile Pro Lys Val
           35                  40                  45
   Ser Glu Thr Thr Lys Leu Leu Asn Leu Gln Arg Asn Asn Phe Pro Val
       50                  55                  60
   Leu Ala Ala Asn Ser Phe Arg Thr Met Pro Asn Leu Val Ser Leu His
   65                  70                  75                  80
   Leu Gln His Cys Asn Ile Arg Glu Val Ala Ala Gly Ala Phe Arg Gly
                   85                  90                  95
   Leu Lys Gln Leu Ile Tyr Leu Tyr Leu Ser His Asn Asp Ile Arg Val
               100                 105                 110
   Leu Arg Ala Gly Ala Phe Asp Asp Leu Thr Glu Leu Thr Tyr Leu Tyr
           115                 120                 125
   Leu Asp His Asn Lys Val Ser Glu Leu Pro Arg Gly Leu Leu Ser Pro
       130                 135                 140
   Leu Val Asn Leu Phe Ile Leu Gln Leu Asn Asn Asn Lys Ile Arg Glu
   145                 150                 155                 160
   Leu Arg Ala Gly Ala Phe Gln Gly Ala Lys Asp Leu Arg Trp Leu Tyr
                   165                 170                 175
   Leu Ser Glu Asn Ala Leu Ser Ser Leu Gln Pro Gly Ser Leu Asp Asp
               180                 185                 190
   Val Glu Asn Leu Ala Lys Phe His Leu Asp Lys Asn Gln Leu Ser Ser
           195                 200                 205
   Tyr Pro Ser Ala Ala Leu Ser Lys Leu Arg Val Val Glu Glu Leu Lys
       210                 215                 220
   Leu Ser His Asn Pro Leu Lys Ser Ile Pro Asp Asn Ala Phe Gln Ser
   225                 230                 235                 240
   Phe Gly Arg Tyr Leu Glu Thr Leu Trp Leu Asp Asn Thr Asn Leu Glu
                   245                 250                 255
   Lys Phe Ser Asp Ala Ala Phe Ser Gly Val Thr Thr Leu Lys His Val
               260                 265                 270
   His Leu Asp Asn Asn Arg Leu Asn Gln Leu Pro Ser Ser Phe Pro Phe
           275                 280                 285
   Asp Asn Leu Glu Thr Leu Thr Leu Thr Asn Asn Pro Trp Lys Cys Thr
       290                 295                 300
   Cys Gln Leu Arg Gly Leu Arg Arg Trp Leu Glu Ala Lys Ala Ser Arg
   305 310 315 320
   Pro Asp Ala Thr Cys Ser Ser Pro Ala Lys Phe Lys Gly Gln Arg Ile
   325 330 335
   Arg Asp Thr Asp Ala Leu Arg Ser Cys Lys Ser Pro Thr Lys Arg Ser
   340 345 350
   Lys Lys Ala Gly Arg His
   355
<210> 43
   <211> 359
   <212> PRT
   <213> Homo sapiens
<400> 43
   Met Val Arg Pro Met Leu Leu Leu Ser Leu Gly Leu Leu Ala Gly Leu
   1               5                   10                  15
   Leu Pro Ala Leu Ala Ala Cys Pro Gln Asn Cys His Cys His Ser Asp
               20                  25                  30
   Leu Gln His Val Ile Cys Asp Lys Val Gly Leu Gln Lys Ile Pro Lys
           35                  40                  45
   Val Ser Glu Lys Thr Lys Leu Leu Asn Leu Gln Arg Asn Asn Phe Pro
       50                  55                  60
   Val Leu Ala Ala Asn Ser Phe Arg Ala Met Pro Asn Leu Val Ser Leu
   65                  70                  75                  80
   His Leu Gln His Cys Gln Ile Arg Glu Val Ala Ala Gly Ala Phe Arg
                   85                  90                  95
   Gly Leu Lys Gln Leu Ile Tyr Leu Tyr Leu Ser His Asn Asp Ile Arg
               100                 105                 110
   Val Val Arg Ala Gly Ala Phe Asp Asp Leu Thr Glu Leu Thr Tyr Leu
           115                 120                 125
   Tyr Leu Asp His Asn Lys Val Thr Glu Leu Pro Arg Gly Leu Leu Ser
       130                 135                 140
   Pro Leu Val Asn Leu Phe Ile Leu Gln Leu Asn Asn Asn Lys Ile Arg
   145                 150                 155                 160
   Glu Leu Arg Ala Gly Pro Phe Gln Gly Ala Lys Asp Leu Arg Trp Leu
   165 170 175
   Tyr Leu Ser Glu Asn Ala Leu Ser Ser Leu Gln Pro Gly Ala Leu Asp
   180 185 190
   Asp Val Glu Asn Leu Ala Lys Phe His Val Asp Arg Asn Gln Leu Ser
   195 200 205
   Ser Tyr Pro Ser Ala Ala Leu Ser Lys Leu Arg Val Val Glu Glu Leu
   210 215 220
   Lys Leu Ser His Asn Pro Leu Lys Ser Ile Pro Asp Asn Ala Phe Gln
   225 230 235 240
   Ser Phe Gly Arg Tyr Leu Glu Thr Leu Trp Leu Asp Asn Thr Asn Leu
   245 250 255
   Glu Lys Phe Ser Asp Gly Ala Phe Leu Gly Val Thr Thr Leu Lys His
   260 265 270
   Val His Leu Glu Asn Asn Arg Leu Asn Gln Leu Pro Ser Asn Phe Pro
   275 280 285
   Phe Asp Ser Leu Glu Thr Leu Ala Leu Thr Asn Asn Pro Trp Lys Cys
   290 295 300
   Thr Cys Gln Leu Arg Gly Leu Arg Arg Trp Leu Glu Ala Lys Ala Ser
   305 310 315 320
   Arg Pro Asp Ala Thr Cys Ala Ser Pro Ala Lys Phe Lys Gly Gln His
   325 330 335
   Ile Arg Asp Thr Asp Ala Phe Arg Ser Cys Lys Phe Pro Thr Lys Arg
   340 345 350
   Ser Lys Lys Ala Gly Arg His
   355
<210> 44
   <211> 16
   <212> PRT
   <213> artificial sequence
<220>
   <223> fragment chondroadhérine murine
<400> 44
   Cys Pro Gln Asn Cys His Cys His Gly Asp Leu Gln His Val Ile Cys
   1 5 10 15
<210> 45
   <211> 16
   <212> PRT
   <213> artificial sequence
<220>
   <223> fragment chondroadhérine humaine
<400> 45
   Cys Pro Gln Asn Cys His Cys His Ser Asp Leu Gln His Val Ile Cys
   1 5 10 15
<210> 46
   <211> 35
   <212> PRT
   <213> artificial sequence
<220>
   <223> 12-46 chondroadhérine murine
<400> 46
   Leu Ala Ile Leu Leu Pro Ala Leu Ala Ala Cys Pro Gln Asn Cys His
   1 5 10 15
   Cys His Gly Asp Leu Gln His Val Ile Cys Asp Lys Val Gly Leu Gln
   20 25 30
   Lys Ile Pro
   35
<210> 47
   <211> 35
   <212> PRT
   <213> artificial sequence
<220>
   <223> 13-47 chondroadhérine humaine
<400> 47
   Leu Ala Gly Leu Leu Pro Ala Leu Ala Ala Cys Pro Gln Asn Cys His
   1 5 10 15
   Cys His Ser Asp Leu Gln His Val Ile Cys Asp Lys Val Gly Leu Gln
   20 25 30
   Lys Ile Pro
   35
<210> 48
   <211> 476
   <212> PRT
   <213> Mus musculus
<400> 48
   Met Leu Ile Leu Leu Leu His Ala Val Val Phe Ser Leu Pro Tyr Thr
   1               5                   10                  15
   Arg Ala Thr Glu Ala Cys Leu Arg Ala Cys Pro Ala Ala Cys Thr Cys
               20                  25                  30
   Ser His Val Glu Arg Gly Cys Ser Val Arg Cys Asp Arg Ala Gly Leu
           35                  40                  45
   Gln Arg Val Pro Gln Glu Phe Pro Cys Glu Ala Ala Ser Ile Asp Leu
       50                  55                  60
   Asp Arg Asn Gly Leu Arg Ile Leu Gly Glu Arg Ala Phe Gly Thr Leu
   65                  70                  75                  80
   Pro Ser Leu Arg Arg Leu Ser Leu Arg His Asn Asn Leu Ser Phe Ile
                   85                  90                  95
   Thr Pro Gly Ala Phe Lys Gly Leu Pro Arg Leu Ala Glu Leu Arg Leu
               100                 105                 110
   Ala His Asn Gly Glu Leu Arg Tyr Leu His Val Arg Thr Phe Ala Ala
           115                 120                 125
   Leu Gly Arg Leu Arg Arg Leu Asp Leu Ala Ala Cys Arg Leu Phe Ser
       130                 135                 140
   Val Pro Glu Arg Leu Leu Ala Glu Leu Pro Ala Leu Arg Glu Leu Thr
   145                 150                 155                 160
   Ala Phe Asp Asn Leu Phe Arg Arg Val Pro Gly Ala Leu Arg Gly Leu
                   165                 170                 175
   Ala Asn Leu Thr His Ala His Phe Glu Arg Ser Arg Ile Glu Ala Val
               180                 185                 190
   Ala Ser Gly Ser Leu Leu Gly Met Arg Arg Leu Arg Ser Leu Ser Leu
           195                 200                 205
   Gln Ala Asn Arg Val Arg Ala Val His Ala Gly Ala Phe Gly Asp Cys
       210                 215                 220
   Gly Ala Leu Glu Asp Leu Leu Leu Asn Asp Asn Leu Leu Ala Thr Leu
   225                 230                 235                 240
   Pro Ala Ala Ala Phe Arg Gly Leu Arg Arg Leu Arg Thr Leu Asn Leu
   245 250 255
   Gly Gly Asn Ala Leu Gly Ser Val Ala Arg Ala Trp Phe Ser Asp Leu
   260 265 270
   Ala Glu Leu Glu Leu Leu Tyr Leu Asp Arg Asn Ser Ile Thr Phe Val
   275 280 285
   Glu Glu Gly Ala Phe Gln Asn Leu Ser Gly Leu Leu Ala Leu His Leu
   290 295 300
   Asn Gly Asn Arg Leu Thr Val Leu Ser Trp Ala Ala Phe Gln Pro Gly
   305 310 315 320
   Phe Phe Leu Gly Arg Leu Phe Leu Phe Arg Asn Pro Trp Arg Cys Asp
   325 330 335
   Cys Gln Leu Glu Trp Leu Arg Asp Trp Met Glu Gly Ser Gly Arg Val
   340 345 350
   Ala Asp Val Ala Cys Ala Ser Pro Gly Ser Val Ala Gly Gln Asp Leu
   355 360 365
   Ser Gln Val Val Phe Glu Arg Ser Ser Asp Gly Leu Cys Val Asp Pro
   370 375 380
   Asp Glu Leu Asn Phe Thr Thr Ser Ser Pro Gly Pro Ser Pro Glu Pro
   385 390 395 400
   Val Ala Thr Thr Val Ser Arg Phe Ser Ser Leu Leu Ser Lys Leu Leu
   405 410 415
   Ala Pro Arg Ala Pro Val Glu Glu Val Ala Asn Thr Thr Trp Glu Leu
   420 425 430
   Val Asn Val Ser Leu Asn Asp Ser Phe Arg Ser His Ala Val Met Val
   435 440 445
   Phe Cys Tyr Lys Ala Thr Phe Leu Phe Thr Ser Cys Val Leu Leu Ser
   450 455 460
   Leu Ala Gln Tyr Val Val Val Gly Leu Gln Arg Glu
   465 470 475
<210> 49
   <211> 481
   <212> PRT
   <213> Homo sapiens
<400> 49
   Met Lys Gly Arg Gly Met Leu Val Leu Leu Leu His Ala Val Val Leu
   1               5                   10                  15
   Gly Leu Pro Ser Ala Trp Ala Val Gly Ala Cys Ala Arg Ala Cys Pro
               20                  25                  30
   Ala Ala Cys Ala Cys Ser Thr Val Glu Arg Gly Cys Ser Val Arg Cys
           35                  40                  45
   Asp Arg Ala Gly Leu Leu Arg Val Pro Ala Glu Leu Pro Cys Glu Ala
       50                  55                  60
   Val Ser Ile Asp Leu Asp Arg Asn Gly Leu Arg Phe Leu Gly Glu Arg
   65                  70                  75                  80
   Ala Phe Gly Thr Leu Pro Ser Leu Arg Arg Leu Ser Leu Arg His Asn
                   85                  90                  95
   Asn Leu Ser Phe Ile Thr Pro Gly Ala Phe Lys Gly Leu Pro Arg Leu
               100                 105                 110
   Ala Glu Leu Arg Leu Ala His Asn Gly Asp Leu Arg Tyr Leu His Ala
           115                 120                 125
   Arg Thr Phe Ala Ala Leu Ser Arg Leu Arg Arg Leu Asp Leu Ala Ala
       130                 135                 140
   Cys Arg Leu Phe Ser Val Pro Glu Arg Leu Leu Ala Glu Leu Pro Ala
   145                 150                 155                 160
   Leu Arg Glu Leu Ala Ala Phe Asp Asn Leu Phe Arg Arg Val Pro Gly
                   165                 170                 175
   Ala Leu Arg Gly Leu Ala Asn Leu Thr His Ala His Leu Glu Arg Gly
               180                 185                 190
   Arg Ile Glu Ala Val Ala Ser Ser Ser Leu Gln Gly Leu Arg Arg Leu
           195                 200                 205
   Arg Ser Leu Ser Leu Gln Ala Asn Arg Val Arg Ala Val His Ala Gly
       210                 215                 220
   Ala Phe Gly Asp Cys Gly Val Leu Glu His Leu Leu Leu Asn Asp Asn
   225                 230                 235                 240
   Leu Leu Ala Glu Leu Pro Ala Asp Ala Phe Arg Gly Leu Arg Arg Leu
                   245                 250                 255
   Arg Thr Leu Asn Leu Gly Gly Asn Ala Leu Asp Arg Val Ala Arg Ala
               260                 265                 270
   Trp Phe Ala Asp Leu Ala Glu Leu Glu Leu Leu Tyr Leu Asp Arg Asn
           275                 280                 285
   Ser Ile Ala Phe Val Glu Glu Gly Ala Phe Gln Asn Leu Ser Gly Leu
       290                 295                 300
   Leu Ala Leu His Leu Asn Gly Asn Arg Leu Thr Val Leu Ala Trp Val
   305                 310                 315                 320
   Ala Phe Gln Pro Gly Phe Phe Leu Gly Arg Leu Phe Leu Phe Arg Asn
                   325                 330                 335
   Pro Trp Cys Cys Asp Cys Arg Leu Glu Trp Leu Arg Asp Trp Met Glu
               340                 345                 350
   Gly Ser Gly Arg Val Thr Asp Val Pro Cys Ala Ser Pro Gly Ser Val
           355                 360                 365
   Ala Gly Leu Asp Leu Ser Gln Val Thr Phe Gly Arg Ser Ser Asp Gly
       370                 375                 380
   Leu Cys Val Asp Pro Glu Glu Leu Asn Leu Thr Thr Ser Ser Pro Gly
   385                 390                 395                 400
   Pro Ser Pro Glu Pro Ala Ala Thr Thr Val Ser Arg Phe Ser Ser Leu
                   405                 410                 415
   Leu Ser Lys Leu Leu Ala Pro Arg Val Pro Val Glu Glu Ala Ala Asn
               420                 425                 430
   Thr Thr Gly Gly Leu Ala Asn Ala Ser Leu Ser Asp Ser Leu Ser Ser
           435                 440                 445
   Arg Gly Val Gly Gly Ala Gly Arg Gln Pro Trp Phe Leu Leu Ala Ser
       450                 455                 460
   Cys Leu Leu Pro Ser Val Ala Gln His Val Val Phe Gly Leu Gln Met
   465                 470                 475                 480
   Asp
<210> 50
   <211> 22
   <212> PRT
   <213> artificial sequence
<220>
   <223> fragment nyctalopine murine
<400> 50
   Cys Leu Arg Ala Cys Pro Ala Ala Cys Thr Cys Ser His Val Glu Arg
   1               5                   10                  15
   Gly Cys Ser Val Arg Cys
               20
<210> 51
   <211> 22
   <212> PRT
   <213> artificial sequence
<220>
   <223> fragment nyctalopine humaine
<400> 51
   Cys Ala Arg Ala Cys Pro Ala Ala Cys Ala Cys Ser Thr Val Glu Arg
   1               5                   10                  15
   Gly Cys Ser Val Arg Cys
               20
<210> 52
   <211> 38
   <212> PRT
   <213> artificial sequence
<220>
   <223> 15-52 nyctalopine murine
<400> 52
   Tyr Thr Arg Ala Thr Glu Ala Cys Leu Arg Ala Cys Pro Ala Ala Cys
   1               5                   10                  15
   Thr Cys Ser His Val Glu Arg Gly Cys Ser Val Arg Cys Asp Arg Ala
               20                  25                  30
   Gly Leu Gln Arg Val Pro
           35
<210> 53
   <211> 38
   <212> PRT
   <213> artificial sequence
<220>
   <223> 20-57 nyctalopine humaine
<400> 53
   Ser Ala Trp Ala Val Gly Ala Cys Ala Arg Ala Cys Pro Ala Ala Cys
   1               5                   10                  15
   Ala Cys Ser Thr Val Glu Arg Gly Cys Ser Val Arg Cys Asp Arg Ala
               20                  25                  30
   Gly Leu Leu Arg Val Pro
           35
<210> 54
   <211> 354
   <212> PRT
   <213> Mus musculus
<400> 54
   Met Leu Cys Ser Leu Phe Leu Leu Leu Leu Ala Val Gly Arg Val Gln
   1               5                   10                  15
   Thr Thr Arg Pro Cys Phe Pro Gly Cys Gln Cys Glu Glu Glu Thr Phe
               20                  25                  30
   Gly Leu Phe Asp Ser Phe Ser Leu Ile Arg Val Asp Cys Ser Ser Leu
           35                  40                  45
   Gly Pro His Ile Val Pro Val Pro Ile Pro Leu Asp Thr Ala His Leu
       50                  55                  60
   Asp Leu Ser Ser Asn Arg Leu Glu Thr Val Asn Glu Ser Val Leu Ala
   65                  70                  75                  80
   Gly Pro Gly Tyr Thr Thr Leu Ala Gly Leu Asp Leu Ser Tyr Asn Leu
                   85                  90                  95
   Leu Thr Ser Ile Met Pro Ser Ala Phe Ser Arg Leu Arg Tyr Leu Glu
               100                 105                 110
   Ser Leu Asp Leu Ser His Asn Gly Leu Ala Ala Leu Pro Ala Glu Ile
           115                 120                 125
   Phe Thr Ser Ser Pro Leu Ser Asp Ile Asn Leu Ser His Asn Arg Leu
       130                 135                 140
   Arg Glu Val Ser Ile Ser Ala Phe Thr Thr His Ser Gln Gly Arg Ala
   145                 150                 155                 160
   Leu His Val Asp Leu Ser His Asn Leu Ile His Arg Leu Leu Pro His
                   165                 170                 175
   Pro Ala Arg Ala Ser Leu Pro Ala Pro Thr Ile Gln Ser Leu Asn Leu
               180                 185                 190
   Ser Trp Asn Arg Phe Arg Ala Val Pro Asp Leu Arg Asp Leu Pro Leu
           195                 200                 205
   Arg Tyr Leu Ser Leu Asp Gly Asn Pro Leu Ala Thr Ile Asn Pro Asp
       210                 215                 220
   Ala Phe Met Gly Leu Ala Gly Leu Thr His Leu Ser Leu Ala Ser Leu
   225                 230                 235                 240
   Gln Gly Ile Leu His Leu Pro Pro His Gly Phe Arg Glu Leu Pro Gly
                   245                 250                 255
   Leu Gln Val Leu Asp Leu Ser Gly Asn Pro Lys Leu Lys Trp Ala Gly
               260                 265                 270
   Ala Glu Val Phe Ser Gly Leu Gly Leu Leu Gln Glu Leu Asp Leu Ser
           275                 280                 285
   Gly Ser Ser Leu Val Pro Leu Pro Glu Met Leu Leu His His Leu Pro
       290                 295                 300
   Ala Leu Gln Ser Val Ser Val Gly Gln Asp Val Gln Cys Arg Arg Leu
   305                 310                 315                 320
   Val Arg Glu Gly Ala Tyr His Arg Gln Pro Gly Ser Ser Pro Lys Val
                   325                 330                 335
   Val Leu His Cys Gly Asp Thr Gln Glu Ser Ala Ala Arg Gly Pro Asp
               340                 345                 350
   Ile Leu
<210> 55
   <211> 353
   <212> PRT
   <213> Homo sapiens
<400> 55
   Met Pro Trp Pro Leu Leu Leu Leu Leu Ala Val Ser Gly Ala Gln Thr
   1               5                   10                  15
   Thr Arg Pro Cys Phe Pro Gly Cys Gln Cys Glu Val Glu Thr Phe Gly
               20                  25                  30
   Leu Phe Asp Ser Phe Ser Leu Thr Arg Val Asp Cys Ser Gly Leu Gly
           35                  40                  45
   Pro His Ile Met Pro Val Pro Ile Pro Leu Asp Thr Ala His Leu Asp
       50                  55                  60
   Leu Ser Ser Asn Arg Leu Glu Met Val Asn Glu Ser Val Leu Ala Gly
   65                  70                  75                  80
   Pro Gly Tyr Thr Thr Leu Ala Gly Leu Asp Leu Ser His Asn Leu Leu
                   85                  90                  95
   Thr Ser Ile Ser Pro Thr Ala Phe Ser Arg Leu Arg Tyr Leu Glu Ser
               100                 105                 110
   Leu Asp Leu Ser His Asn Gly Leu Thr Ala Leu Pro Ala Glu Ser Phe
           115                 120                 125
   Thr Ser Ser Pro Leu Ser Asp Val Asn Leu Ser His Asn Gln Leu Arg
       130                 135                 140
   Glu Val Ser Val Ser Ala Phe Thr Thr His Ser Gln Gly Arg Ala Leu
   145                 150                 155                 160
   His Val Asp Leu Ser His Asn Leu Ile His Arg Leu Val Pro His Pro
                   165                 170                 175
   Thr Arg Ala Gly Leu Pro Ala Pro Thr Ile Gln Ser Leu Asn Leu Ala
               180                 185                 190
   Trp Asn Arg Leu His Ala Val Pro Asn Leu Arg Asp Leu Pro Leu Arg
           195                 200                 205
   Tyr Leu Ser Leu Asp Gly Asn Pro Leu Ala Val Ile Gly Pro Gly Ala
       210                 215                 220
   Phe Ala Gly Leu Gly Gly Leu Thr His Leu Ser Leu Ala Ser Leu Gln
   225                 230                 235                 240
   Arg Leu Pro Glu Leu Ala Pro Ser Gly Phe Arg Glu Leu Pro Gly Leu
                   245                 250                 255
   Gln Val Leu Asp Leu Ser Gly Asn Pro Lys Leu Asn Trp Ala Gly Ala
   260 265 270
   Glu Val Phe Ser Gly Leu Ser Ser Leu Gln Glu Leu Asp Leu Ser Gly
   275 280 285
   Thr Asn Leu Val Pro Leu Pro Glu Ala Leu Leu Leu His Leu Pro Ala
   290 295 300
   Leu Gln Ser Val Ser Val Gly Gln Asp Val Arg Cys Arg Arg Leu Val
   305 310 315 320
   Arg Glu Gly Thr Tyr Pro Arg Arg Pro Gly Ser Ser Pro Lys Val Ala
   325 330 335
   Leu His Cys Val Asp Thr Arg Asp Ser Ala Ala Arg Gly Pro Thr Ile
   340 345 350
   Leu
<210> 56
   <211> 25
   <212> PRT
   <213> artificial sequence
<220>
   <223> fragment tsukushi murin
<400> 56
   Cys Phe Pro Gly Cys Gln Cys Glu Glu Glu Thr Phe Gly Leu Phe Asp
   1 5 10 15
   Ser Phe Ser Leu Ile Arg Val Asp Cys
   20 25
<210> 57
   <211> 24
   <212> PRT
   <213> artificial sequence
<220>
   <223> fragment tsukushi humain
<400> 57
   Cys Phe Pro Gly Cys Gln Cys Glu Val Glu Thr Phe Gly Leu Phe Asp
   1 5 10 15
   Ser Phe Ser Leu Thr Arg Val Asp
   20
<210> 58
   <211> 41
   <212> PRT
   <213> artificial sequence
<220>
   <223> 14-54 tsukushi murin
<400> 58
   Arg Val Gln Thr Thr Arg Pro Cys Phe Pro Gly Cys Gln Cys Glu Glu
   1               5                   10                  15
   Glu Thr Phe Gly Leu Phe Asp Ser Phe Ser Leu Ile Arg Val Asp Cys
               20                  25                  30
   Ser Ser Leu Gly Pro His Ile Val Pro
           35                  40
<210> 59
   <211> 41
   <212> PRT
   <213> artificial sequence
<220>
   <223> 13-53 tsukushi humain
<400> 59
   Gly Ala Gln Thr Thr Arg Pro Cys Phe Pro Gly Cys Gln Cys Glu Val
   1               5                   10                  15
   Glu Thr Phe Gly Leu Phe Asp Ser Phe Ser Leu Thr Arg Val Asp Cys
               20                  25                  30
   Ser Gly Leu Gly Pro His Ile Met Pro
           35                  40
<210> 60
   <211> 611
   <212> PRT
   <213> Mus musculus
<400> 60
   Met Ala Gly Ser Arg Gly Leu Pro Leu Leu Leu Leu Val Leu Gln Leu
   1               5                   10                  15
   Phe Leu Gly Pro Val Leu Pro Val Arg Ala Pro Val Phe Gly Arg Ser
               20                  25                  30
   Asp Thr Pro Thr Leu Ser Pro Glu Glu Asn Glu Phe Val Glu Glu Glu
           35                  40                  45
   Asn Gln Pro Val Leu Val Leu Ser Ser Glu Glu Pro Glu Pro Gly Pro
       50                  55                  60
   Ala Thr Val Asp Cys Pro Arg Asp Cys Ala Cys Ser Gln Glu Gly Val
   65                  70                  75                  80
   Val Asp Cys Gly Gly Ile Asp Leu Arg Glu Phe Pro Gly Asp Leu Pro
                   85                  90                  95
   Glu His Thr Asn His Leu Ser Leu Gln Asn Asn Gln Leu Glu Lys Ile
               100                 105                 110
   Tyr Pro Glu Glu Leu Ser Arg Leu Gln Arg Leu Glu Thr Leu Asn Leu
           115                 120                 125
   Gln Asn Asn Arg Leu Thr Ser Arg Gly Leu Pro Glu Glu Ala Phe Glu
       130                 135                 140
   His Leu Thr Ser Leu Asn Tyr Leu Tyr Leu Ala Asn Asn Lys Leu Thr
   145                 150                 155                 160
   Leu Ala Pro Arg Phe Leu Pro Asn Ala Leu Ile Ser Val Asp Phe Ala
                   165                 170                 175
   Ala Asn Tyr Leu Thr Lys Ile Tyr Gly Leu Thr Phe Gly Gln Lys Pro
               180                 185                 190
   Asn Leu Arg Ser Val Tyr Leu His Asn Asn Lys Leu Ala Asp Ala Gly
           195                 200                 205
   Leu Pro Asp His Met Phe Asn Gly Ser Ser Asn Val Glu Ile Leu Ile
       210                 215                 220
   Leu Ser Ser Asn Phe Leu Arg His Val Pro Lys His Leu Pro Pro Ala
   225                 230                 235                 240
   Leu Tyr Lys Leu His Leu Lys Asn Asn Lys Leu Glu Lys Ile Pro Pro
                   245                 250                 255
   Gly Ala Phe Ser Glu Leu Ser Asn Leu Arg Glu Leu Tyr Leu Gln Asn
               260                 265                 270
   Asn Tyr Leu Thr Asp Glu Gly Leu Asp Asn Glu Thr Phe Trp Lys Leu
           275                 280                 285
   Ser Ser Leu Glu Tyr Leu Asp Leu Ser Ser Asn Asn Leu Ser Arg Val
   290 295 300
   Pro Ala Gly Leu Pro Arg Ser Leu Val Leu Leu His Leu Glu Lys Asn
   305 310 315 320
   Ala Ile Gln Ser Val Glu Ala Asp Val Leu Thr Pro Ile Arg Asn Leu
   325 330 335
   Glu Tyr Leu Leu Leu His Ser Asn Gln Leu Gln Ala Lys Gly Ile His
   340 345 350
   Pro Leu Ala Phe Gln Gly Leu Lys Lys Leu His Thr Val His Leu Tyr
   355 360 365
   Asn Asn Ala Leu Glu Arg Val Pro Ser Gly Leu Pro Arg Arg Val Arg
   370 375 380
   Thr Leu Met Ile Leu His Asn Gln Ile Thr Gly Ile Gly Arg Glu Asp
   385 390 395 400
   Phe Ala Thr Thr Tyr Phe Leu Glu Glu Leu Asn Leu Ser Tyr Asn Arg
   405 410 415
   Ile Thr Ser Pro Gln Met His Arg Asp Ala Phe Arg Lys Leu Arg Leu
   420 425 430
   Leu Arg Ser Leu Asp Leu Ser Gly Asn Arg Leu Gln Thr Leu Pro Pro
   435 440 445
   Gly Leu Pro Lys Asn Val His Val Leu Lys Val Lys Arg Asn Glu Leu
   450 455 460
   Ala Ala Leu Ala Arg Gly Ala Leu Ala Gly Met Ala Gln Leu Arg Glu
   465 470 475 480
   Leu Tyr Leu Thr Gly Asn Arg Leu Arg Ser Arg Ala Leu Gly Pro Arg
   485 490 495
   Ala Trp Val Asp Leu Ala Gly Leu Gln Leu Leu Asp Ile Ala Gly Asn
   500 505 510
   Gln Leu Thr Glu Val Pro Glu Gly Leu Pro Pro Ser Leu Glu Tyr Leu
   515 520 525
   Tyr Leu Gln Asn Asn Lys Ile Ser Ala Val Pro Ala Asn Ala Phe Asp
   530 535 540
   Ser Thr Pro Asn Leu Lys Gly Ile Phe Leu Arg Phe Asn Lys Leu Ala
   545 550 555 560
   Val Gly Ser Val Val Glu Ser Ala Phe Arg Arg Leu Lys His Leu Gln
   565 570 575
   Val Leu Asp Ile Glu Gly Asn Phe Glu Phe Gly Asn Gly Ser Lys Asp
   580 585 590
   Lys Asp Glu Glu Glu Glu Glu Glu Glu Glu Glu Glu Asp Glu Glu Glu
   595 600 605
   Glu Thr Arg
   610
<210> 61
   <211> 613
   <212> PRT
   <213> Homo sapiens
<400> 61
   Met Ala Gln Ser Arg Val Leu Leu Leu Leu Leu Leu Leu Pro Pro Gln
   1 5 10 15
   Leu His Leu Gly Pro Val Leu Ala Val Arg Ala Pro Gly Phe Gly Arg
   20 25 30
   Ser Gly Gly His Ser Leu Ser Pro Glu Glu Asn Glu Phe Ala Glu Glu
   35 40 45
   Glu Pro Val Leu Val Leu Ser Pro Glu Glu Pro Gly Pro Gly Pro Ala
   50 55 60
   Ala Val Ser Cys Pro Arg Asp Cys Ala Cys Ser Gln Glu Gly Val Val
   65 70 75 80
   Asp Cys Gly Gly Ile Asp Leu Arg Glu Phe Pro Gly Asp Leu Pro Glu
   85 90 95
   His Thr Asn His Leu Ser Leu Gln Asn Asn Gln Leu Glu Lys Ile Tyr
   100 105 110
   Pro Glu Glu Leu Ser Arg Leu His Arg Leu Glu Thr Leu Asn Leu Gln
   115 120 125
   Asn Asn Arg Leu Thr Ser Arg Gly Leu Pro Glu Lys Ala Phe Glu His
   130 135 140
   Leu Thr Asn Leu Asn Tyr Leu Tyr Leu Ala Asn Asn Lys Leu Thr Leu
   145                 150                 155                 160
   Ala Pro Arg Phe Leu Pro Asn Ala Leu Ile Ser Val Asp Phe Ala Ala
                   165                 170                 175
   Asn Tyr Leu Thr Lys Ile Tyr Gly Leu Thr Phe Gly Gln Lys Pro Asn
               180                 185                 190
   Leu Arg Ser Val Tyr Leu His Asn Asn Lys Leu Ala Asp Ala Gly Leu
           195                 200                 205
   Pro Asp Asn Met Phe Asn Gly Ser Ser Asn Val Glu Val Leu Ile Leu
       210                 215                 220
   Ser Ser Asn Phe Leu Arg His Val Pro Lys His Leu Pro Pro Ala Leu
   225                 230                 235                 240
   Tyr Lys Leu His Leu Lys Asn Asn Lys Leu Glu Lys Ile Pro Pro Gly
                   245                 250                 255
   Ala Phe Ser Glu Leu Ser Ser Leu Arg Glu Leu Tyr Leu Gln Asn Asn
               260                 265                 270
   Tyr Leu Thr Asp Glu Gly Leu Asp Asn Glu Thr Phe Trp Lys Leu Ser
           275                 280                 285
   Ser Leu Glu Tyr Leu Asp Leu Ser Ser Asn Asn Leu Ser Arg Val Pro
       290                 295                 300
   Ala Gly Leu Pro Arg Ser Leu Val Leu Leu His Leu Glu Lys Asn Ala
   305                 310                 315                 320
   Ile Arg Ser Val Asp Ala Asn Val Leu Thr Pro Ile Arg Ser Leu Glu
                   325                 330                 335
   Tyr Leu Leu Leu His Ser Asn Gln Leu Arg Glu Gln Gly Ile His Pro
               340                 345                 350
   Leu Ala Phe Gln Gly Leu Lys Arg Leu His Thr Val His Leu Tyr Asn
           355                 360                 365
   Asn Ala Leu Glu Arg Val Pro Ser Gly Leu Pro Arg Arg Val Arg Thr
       370                 375                 380
   Leu Met Ile Leu His Asn Gln Ile Thr Gly Ile Gly Arg Glu Asp Phe
   385                 390                 395                 400
   Ala Thr Thr Tyr Phe Leu Glu Glu Leu Asn Leu Ser Tyr Asn Arg Ile
   405 410 415
   Thr Ser Pro Gln Val His Arg Asp Ala Phe Arg Lys Leu Arg Leu Leu
   420 425 430
   Arg Ser Leu Asp Leu Ser Gly Asn Arg Leu His Met Leu Pro Pro Gly
   435 440 445
   Leu Pro Arg Asn Val His Val Leu Lys Val Lys Arg Asn Glu Leu Ala
   450 455 460
   Ala Leu Ala Arg Gly Ala Leu Ala Gly Met Ala Gln Leu Arg Glu Leu
   465 470 475 480
   Tyr Leu Thr Ser Asn Arg Leu Arg Ser Arg Ala Leu Gly Pro Arg Ala
   485 490 495
   Trp Val Asp Leu Ala His Leu Gln Leu Leu Asp Ile Ala Gly Asn Gln
   500 505 510
   Leu Thr Glu Ile Pro Glu Gly Leu Pro Glu Ser Leu Glu Tyr Leu Tyr
   515 520 525
   Leu Gln Asn Asn Lys Ile Ser Ala Val Pro Ala Asn Ala Phe Asp Ser
   530 535 540
   Thr Pro Asn Leu Lys Gly Ile Phe Leu Arg Phe Asn Lys Leu Ala Val
   545 550 555 560
   Gly Ser Val Val Asp Ser Ala Phe Arg Arg Leu Lys His Leu Gln Val
   565 570 575
   Leu Asp Ile Glu Gly Asn Leu Glu Phe Gly Asp Ile Ser Lys Asp Arg
   580 585 590
   Gly Arg Leu Gly Lys Glu Lys Glu Glu Glu Glu Glu Glu Glu Glu Glu
   595 600 605
   Glu Glu Glu Thr Arg
   610
<210> 62
   <211> 15
   <212> PRT
   <213> artificial sequence
<220>
   <223> fragment podocan
<400> 62
   Cys Pro Arg Asp Cys Ala Cys Ser Gln Glu Gly Val Val Asp Cys
   1               5                   10                  15
<210> 63
   <211> 31
   <212> PRT
   <213> artificial sequence
<220>
   <223> 62-92 podocan murin
<400> 63
   Pro Gly Pro Ala Thr Val Asp Cys Pro Arg Asp Cys Ala Cys Ser Gln
   1               5                   10                  15
   Glu Gly Val Val Asp Cys Gly Gly Ile Asp Leu Arg Glu Phe Pro
               20                  25                  30
<210> 64
   <211> 31
   <212> PRT
   <213> artificial sequence
<220>
   <223> 61-91 podocan humain
<400> 64
   Pro Gly Pro Ala Ala Val Ser Cys Pro Arg Asp Cys Ala Cys Ser Gln
   1               5                   10                  15
   Glu Gly Val Val Asp Cys Gly Gly Ile Asp Leu Arg Glu Phe Pro
               20                  25                  30
<210> 65
   <211> 559
   <212> PRT
   <213> Mus musculus
<400> 65
   Met Arg Pro Gln Glu Leu Leu Leu Leu Leu Leu Met Leu Lys Trp Ser
   1               5                   10                  15
   Leu Ala His Thr Glu Asp Pro Ala Phe Pro His Leu Gly Asp Ser Ser
               20                  25                  30
   Gln Pro Leu Pro Arg Pro Cys Pro Trp Arg Cys Ser Cys Pro Arg Asp
           35                  40                  45
   Asp Thr Val Asp Cys Ala Gly Leu Asp Leu Arg Ile Phe Pro Asp Asn
       50                  55                  60
   Ile Thr Arg Ala Ala Arg His Leu Ser Leu Gln Asn Asn Gln Leu Arg
   65                  70                  75                  80
   Glu Leu Pro Tyr Asn Glu Leu Ser Arg Leu Ser Gly Leu Arg Thr Leu
                   85                  90                  95
   Asp Leu His Ser Asn Leu Ile Thr Ser Glu Gly Leu Pro Asp Glu Ala
               100                 105                 110
   Phe Glu Ser Leu Asn Gln Leu Glu Asn Phe Tyr Val Ala His Asn Lys
           115                 120                 125
   Leu Ser Val Ala Pro Gln Phe Leu Pro Arg Ser Leu Arg Val Ala Asp
       130                 135                 140
   Leu Ala Ala Asn Glu Val Val Glu Ile Phe Pro Leu Thr Phe Gly Glu
   145                 150                 155                 160
   Lys Pro Ala Leu Arg Ser Val Tyr Leu His Asn Asn Arg Leu Arg Asn
                   165                 170                 175
   Thr Gly Leu Pro Pro Asn Thr Phe His Gly Ser Glu Val Ile Thr Thr
               180                 185                 190
   Leu Ser Leu Ser Ser Asn Gln Leu Ser Tyr Leu Pro Pro Ser Leu Pro
           195                 200                 205
   Ala Ser Leu Glu Arg Leu His Leu Gln Asn Asn Leu Ile Ser Lys Val
       210                 215                 220
   Pro Arg Gly Ala Leu Ser Leu Gln Thr His Leu Arg Glu Leu Tyr Leu
   225                 230                 235                 240
   Gln His Asn Gln Leu Thr Asp Ser Gly Leu Asp Ala Thr Thr Phe Ser
                   245                 250                 255
   Lys Leu Ser Ser Leu Glu Tyr Leu Asp Leu Ser His Asn Gln Leu Ala
               260                 265                 270
   Thr Val Pro Glu Gly Leu Pro Gly Thr Leu Thr Ile Leu His Leu Gly
           275                 280                 285
   Arg Asn Cys Ile Arg His Val Glu Ala Val Arg Leu His Lys Ala Arg
       290                 295                 300
   Gly Leu Arg Tyr Leu Leu Leu Gln His Asn Lys Leu Gly Ala Ser Ala
   305                 310                 315                 320
   Leu Pro Lys Gly Thr Leu Arg Pro Leu Arg Ala Leu His Thr Leu His
                   325                 330                 335
   Leu Tyr Gly Asn Lys Leu Glu Arg Val Pro Pro Ala Leu Pro Arg His
               340                 345                 350
   Leu Gln Ala Leu Val Met Pro His Asn His Val Ala Ala Leu Gly Ala
           355                 360                 365
   Arg Asp Leu Val Ser Ala Arg Ala Leu Ala Glu Leu Asn Leu Ala Tyr
       370                 375                 380
   Asn Ser Leu Ala Ser Ala His Val His Pro Ser Ala Phe Arg Arg Leu
   385                 390                 395                 400
   Arg Ala Leu Arg Ser Leu Asp Leu Ala Gly Asn Gln Leu Thr Arg Leu
                   405                 410                 415
   Pro Glu Gly Leu Pro Ala Ser Leu Arg Ser Leu Arg Leu Gln Arg Asn
               420                 425                 430
   Gln Leu Arg Thr Leu Glu Pro Glu Gln Leu Ala Gly Leu Asn Lys Leu
           435                 440                 445
   Arg Glu Leu Asn Leu Ala His Asn Arg Leu Arg Val Gly Asp Ile Gly
       450                 455                 460
   Pro Gly Thr Trp His Glu Leu Gln Ala Leu Lys Val Leu Asp Leu Ser
   465                 470                 475                 480
   His Asn Glu Leu Ser Phe Val Pro Pro Asp Leu Pro Glu Ala Leu Glu
                   485                 490                 495
   Glu Leu Tyr Leu Gln Ala Asn Arg Ile Ser His Val Gly Pro Glu Ala
               500                 505                 510
   Phe Leu Ser Thr Pro His Leu Arg Ala Leu Phe Leu Arg Ala Asn Arg
           515                 520                 525
   Leu His Met Thr Ser Ile Arg Ala Glu Ala Leu Gln Gly Leu Thr His
       530                 535                 540
   Leu Arg Val Val Asp Thr Ala Glu Asn Pro Glu Gln Val Leu Val
   545                 550                 555
<210> 66
   <211> 512
   <212> PRT
   <213> Homo sapiens
<400> 66
   Met Ala Glu Ser Gly Leu Ala Met Trp Pro Ser Leu Leu Leu Leu Leu
   1               5                   10                  15
   Leu Leu Pro Gly Pro Pro Pro Val Ala Gly Leu Glu Asp Ala Ala Phe
               20                  25                  30
   Pro His Leu Gly Glu Ser Leu Gln Pro Leu Leu Arg Ala Cys Pro Leu
           35                  40                  45
   Arg Cys Ser Cys Pro Arg Val Asp Thr Val Asp Cys Asp Gly Leu Asp
       50                  55                  60
   Leu Arg Val Phe Pro Asp Asn Ile Thr Arg Ala Ala Gln His Leu Ser
   65                  70                  75                  80
   Leu Gln Asn Asn Gln Leu Gln Glu Leu Pro Tyr Asn Glu Leu Ser Arg
                   85                  90                  95
   Leu Ser Gly Leu Arg Thr Leu Asn Leu His Asn Asn Leu Ile Ser Ser
               100                 105                 110
   Glu Gly Leu Pro Asp Glu Ala Phe Glu Ser Leu Thr Gln Leu Gln His
           115                 120                 125
   Leu Cys Val Ala His Asn Lys Leu Ser Val Ala Pro Gln Phe Leu Pro
       130                 135                 140
   Arg Ser Leu Arg Val Ala Asp Leu Ala Ala Asn Gln Val Met Glu Ile
   145                 150                 155                 160
   Phe Pro Leu Thr Phe Gly Glu Lys Pro Ala Leu Arg Ser Val Tyr Leu
                   165                 170                 175
   His Asn Asn Gln Leu Ser Asn Ala Gly Leu Pro Pro Asp Ala Phe Arg
               180                 185                 190
   Gly Ser Glu Ala Ile Ala Thr Leu Ser Leu Ser Asn Asn Gln Leu Ser
           195                 200                 205
   Tyr Leu Pro Pro Ser Leu Pro Pro Ser Leu Glu Arg Leu His Leu Gln
       210                 215                 220
   Asn Asn Leu Ile Ser Lys Val Pro Arg Gly Ala Leu Ser Arg Gln Thr
   225                 230                 235                 240
   Gln Leu Arg Glu Leu Tyr Leu Gln His Asn Gln Leu Thr Asp Ser Gly
                   245                 250                 255
   Leu Asp Ala Thr Thr Phe Ser Lys Leu His Ser Leu Glu Tyr Leu Asp
               260                 265                 270
   Leu Ser His Asn Gln Leu Thr Thr Val Pro Ala Gly Leu Pro Arg Thr
           275                 280                 285
   Leu Ala Ile Leu His Leu Gly Arg Asn Arg Ile Arg Gln Val Glu Ala
       290                 295                 300
   Ala Arg Leu His Gly Ala Arg Gly Leu Arg Tyr Leu Leu Leu Gln His
   305                 310                 315                 320
   Asn Gln Leu Gly Ser Ser Gly Leu Pro Ala Gly Ala Leu Arg Pro Leu
                   325                 330                 335
   Arg Gly Leu His Thr Leu His Leu Tyr Gly Asn Gly Leu Asp Arg Val
               340                 345                 350
   Pro Pro Ala Leu Pro Arg Arg Leu Arg Ala Leu Val Leu Pro His Asn
           355                 360                 365
   His Val Ala Ala Leu Gly Ala Arg Asp Leu Val Ala Thr Pro Gly Leu
       370                 375                 380
   Thr Glu Leu Asn Leu Ala Tyr Asn Arg Leu Ala Ser Ala Arg Val His
   385                 390                 395                 400
   His Arg Ala Phe Arg Arg Leu Arg Ala Leu Arg Ser Leu Asp Leu Ala
                   405                 410                 415
   Gly Asn Gln Leu Thr Arg Leu Pro Met Gly Leu Pro Thr Gly Leu Arg
               420                 425                 430
   Thr Leu Gln Leu Gln Arg Asn Gln Leu Arg Met Leu Glu Pro Glu Pro
           435                 440                 445
   Leu Ala Gly Leu Asp Gln Leu Arg Glu Leu Ser Leu Ala His Asn Arg
       450                 455                 460
   Leu Arg Val Gly Asp Ile Gly Pro Gly Thr Trp His Glu Leu Gln Ala
   465 470 475 480
   Leu Gln Val Arg His Arg Leu Val Ser His Thr Val Pro Arg Ala Pro
   485 490 495
   Pro Ser Pro Cys Leu Pro Cys His Val Pro Asn Ile Leu Val Ser Trp
   500 505 510
<210> 67
   <211> 15
   <212> PRT
   <213> artificial sequence
<220>
   <223> fragment podocan-like protein 1 murin
<400> 67
   Cys Pro Trp Arg Cys Ser Cys Pro Arg Asp Asp Thr Val Asp Cys
   1 5 10 15
<210> 68
   <211> 15
   <212> PRT
   <213> artificial sequence
<220>
   <223> fragment podocan-like protein 1 humain
<400> 68
   Cys Pro Leu Arg Cys Ser Cys Pro Arg Val Asp Thr Val Asp Cys
   1 5 10 15
<210> 69
   <211> 34
   <212> PRT
   <213> artificial sequence
<220>
   <223> 29-62 podocan-like protein 1 murin
<400> 69
   Gly Asp Ser Ser Gln Pro Leu Pro Arg Pro Cys Pro Trp Arg Cys Ser
   1 5 10 15
   Cys Pro Arg Asp Asp Thr Val Asp Cys Ala Gly Leu Asp Leu Arg Ile
   20 25 30
   Phe Pro
<210> 70
   <211> 34
   <212> PRT
   <213> artificial sequence
<220>
   <223> 36-69 podocan-like protein 1 humain
<400> 70
   Gly Glu Ser Leu Gln Pro Leu Leu Arg Ala Cys Pro Leu Arg Cys Ser
   1               5                   10                  15
   Cys Pro Arg Val Asp Thr Val Asp Cys Asp Gly Leu Asp Leu Arg Val
               20                  25                  30
   Phe Pro
<210> 71
   <211> 670
   <212> PRT
   <213> Mus musculus
<400> 71
   Met Lys Leu Ala Val Leu Phe Cys Phe Ile Leu Leu Ile Val Leu Gln
   1               5                   10                  15
   Thr Asp Cys Glu Arg Gly Thr Arg Arg Gln Arg Arg Arg Met His Gln
               20                  25                  30
   Arg Arg Leu Arg Lys Ser Ser Ser Phe His Leu Arg Ala Asn Arg Gln
           35                  40                  45
   Leu Glu Val Gln Gln Thr Thr Ala Ala Pro Asp Ala Arg Leu Pro Thr
       50                  55                  60
   Ala Asn Ser Asp Tyr Ser Val Glu Glu Asn Ile Glu Ser Leu Leu Ser
   65                  70                  75                  80
   Asn Leu Gly Val Glu Ser Ser Tyr Ser Val Leu Pro Gly Lys Lys Gly
                   85                  90                  95
   Tyr Cys Phe Val Lys Gly Met Ile Met Tyr Asn Lys Ala Val Trp Ser
               100                 105                 110
   Pro Glu Pro Cys Thr Thr Cys Leu Cys Ser Asn Gly Arg Val Leu Cys
           115                 120                 125
   Asp Glu Thr Glu Cys His Pro Lys Ala Cys Pro Tyr Thr Ile Lys Pro
       130                 135                 140
   Glu Gly Glu Cys Cys Pro Ile Cys Ser Asp Ala Glu Gln Glu Ser Ile
   145                 150                 155                 160
   Asn Lys Leu His Lys Gln Val Pro Pro Pro Gln Met Glu Met Asp Gln
                   165                 170                 175
   Val Ala Ile Lys Glu Ala Leu Gln Ser Glu Glu Asp Glu Glu Ile Ala
               180                 185                 190
   Glu Gly His Lys Glu His Lys Lys Glu Thr Ser Val Pro Thr Lys Ile
           195                 200                 205
   His Gly Asp Gly Glu Arg Thr Glu Arg Lys Leu Arg Pro Glu Lys Glu
       210                 215                 220
   Gly Arg Ser Ala His Gln Pro Leu Tyr His Gly Arg Arg Glu Glu Glu
   225                 230                 235                 240
   Glu Ser Lys Glu Glu Thr Glu Arg Glu Gly Glu Glu Glu Glu Glu Glu
                   245                 250                 255
   Glu Glu Glu Glu Glu Glu Asp Ala Ile Arg Gly Asp Val Phe Arg Met
               260                 265                 270
   Ser Ser Arg Val Ile Pro Gly Thr Pro Arg Gly Arg Pro Arg Leu Pro
           275                 280                 285
   Arg Ser Cys Ser Leu Ser Tyr Arg Thr Ile Ser Cys Val His Ala Asp
       290                 295                 300
   Phe Thr Glu Ile Pro Pro Ile Thr Ala Pro Glu Val Thr Asn Leu Glu
   305                 310                 315                 320
   Leu Val Gly Asn Ser Ile Ile Ser Ile Pro Asp Glu Ala Phe Asn Gly
                   325                 330                 335
   Leu Pro Asn Leu Glu Arg Leu Asp Leu Ser Arg Asn Asn Ile Thr Ser
               340                 345                 350
   Ser Gly Ile Gly Pro Lys Ala Phe Lys Ser Leu Lys Lys Leu Met Arg
           355                 360                 365
   Leu Asn Met Asp Gly Asn Asn Leu Val His Ile Pro Ser Asp Leu Pro
       370                 375                 380
   Ser Thr Leu Glu Glu Leu Lys Ile Asn Asp Asn Asn Leu Gln Ala Ile
   385                 390                 395                 400
   Asp Glu Lys Ser Leu Ser Asp Leu Asn Gln Leu Val Thr Leu Glu Leu
   405 410 415
   Glu Gly Asn Asn Leu Ser Glu Ile Asn Val Asp Pro Leu Ala Phe Gln
   420 425 430
   Ser Leu Glu Ser Leu Ser Tyr Leu Arg Leu Gly Arg Asn Lys Phe Arg
   435 440 445
   Ile Ile Pro Gln Gly Leu Pro Ala Ser Thr Glu Glu Leu Tyr Leu Glu
   450 455 460
   Asn Asn Gln Ile Glu Glu Ile Thr Glu Ile Cys Phe Asn His Thr Arg
   465 470 475 480
   Lys Ile Thr Met Ile Ile Leu Arg Tyr Asn Lys Ile Glu Glu Ser Arg
   485 490 495
   Ile Ala Pro Leu Ala Trp Ile Asn Gln Glu Asn Leu Glu Ser Ile Asp
   500 505 510
   Leu Ser Tyr Asn Lys Leu Tyr His Val Pro Ser Tyr Leu Pro Lys Ser
   515 520 525
   Leu Leu His Leu Val Leu Ile Gly Asn Gln Ile Asp Arg Ile Pro Gly
   530 535 540
   Tyr Val Phe Gly His Met Gln Pro Gly Leu Glu Tyr Leu Tyr Leu Ser
   545 550 555 560
   Phe Asn Arg Leu Ser Asp Asp Gly Val Asp Leu Val Ser Phe Tyr Gly
   565 570 575
   Ala Tyr His Ser Leu Arg Glu Leu Phe Leu Asp His Asn Asp Phe Lys
   580 585 590
   Ser Ile Pro Pro Gly Ile Gln Asp Met Lys Ala Leu His Phe Leu Arg
   595 600 605
   Leu Asn Asn Asn Lys Ile Arg Asn Ile His Pro Glu Gln Ile Cys Asn
   610 615 620
   Ala Glu Glu Asp Glu Asp Ser Ala Leu Glu His Leu His Leu Glu Asn
   625 630 635 640
   Asn Tyr Ile Arg Thr Arg Glu Ile Ser Ser Tyr Ala Phe Ser Cys Ile
   645 650 655
   Arg Leu Tyr Ser Ser Ile Val Leu Lys Pro Gln His Ile Lys
   660 665 670
<210> 72
   <211> 677
   <212> PRT
   <213> Homo sapiens
<400> 72
<210> 73
   <211> 31
   <212> PRT
   <213> artificial sequence
<220>
   <223> 109-139 ECM2 murine
<400> 73
   Ala Val Trp Ser Pro Glu Pro Cys Thr Thr Cys Leu Cys Ser Asn Gly
   1               5                   10                  15
   Arg Val Leu Cys Asp Glu Thr Glu Cys His Pro Lys Ala Cys Pro
               20                  25                  30
<210> 74
   <211> 31
   <212> PRT
   <213> artificial sequence
<220>
   <223> 114-144 ECM2 humaine.
<400> 74
   Ala Val Trp Ser Pro Glu Pro Cys Thr Thr Cys Leu Cys Ser Asp Gly
   1               5                   10                  15
   Arg Val Leu Cys Asp Glu Thr Met Cys His Pro Gln Arg Cys Pro
               20                  25                  30
<210> 75
   <211> 41
   <212> PRT
   <213> artificial sequence
<220>
   <223> fragment 31-71 décorine murine
<400> 75
   Asp Glu Ala Ser Gly Ile Ile Pro Tyr Asp Pro Asp Asn Pro Leu Ile
   1               5                   10                  15
   Ser Met Cys Pro Tyr Arg Cys Gln Cys His Leu Arg Val Val Gln Cys
               20                  25                  30
   Ser Asp Leu Gly Leu Asp Lys Val Pro
           35                  40